# EUROPEAN PATENT APPLICATION

(11) **EP 3 329 905 A1**
(43) Date of publication of application: **06.06.2018**
(21) Application number: 16202306.3
(22) Date of filing: 05.12.2016
(51) Int. Cl.: A61K 8/64, A61K 38/00, A61Q 19/08

(54) **TOPICAL COSMETIC COMPOSITIONS COMPRISING AN OLIGOPEPTIDE AGAINST ANTI-AGING OF THE SKIN**

(71) Applicant: Nuritas Limited, D2 Dublin (IE)
(72) Inventor: The designation of the inventor has not yet been filed
(74) Representative: Cahill, Susanne

(57) **Abstract**

A topical cosmetic composition comprising a cosmetically or pharmaceutically effective amount of a peptide comprising an amino acid sequence of SEQUENCE ID NO. 1 or a variant thereof.

SEQUENCE LISTING

<110>  Nuritas

<120>  Composition

<130>  P12182EP01

<160>  85

<170>  PatentIn version 3.5

<210>  1
<211>  11
<212>  PRT
<213>  Artificial Sequence

<220>
<223>  Peptide

<400>  1

Trp Lys Asp Glu Ala Gly Lys Pro Leu Val Lys
1               5                   10

<210>  2
<211>  11
<212>  PRT
<213>  Artificial Sequence

<220>
<223>  Peptide

<400>  2

Trp Lys Glu Glu Ala Gly Lys Pro Leu Val Lys
1               5                   10

<210>  3
<211>  11
<212>  PRT
<213>  Artificial Sequence

<220>
<223>  Peptide

<400>  3

Phe Lys Asp Glu Ala Gly Lys Pro Leu Val Lys
1               5                   10

<210>  4
<211>  11
<212>  PRT
<213>  Artificial Sequence

<220>
<223>  Peptide

<400>  4

Trp Lys Asp Glu Ala Gly Lys Pro Met Val Lys
1               5                   10

<210>  5
<211>  11
<212>  PRT
<213>  Artificial Sequence

<220>
<223>  Peptide

<400>  5

Trp Lys Asp Glu Ala Gly Arg Pro Leu Val Lys
1               5                   10

<210>  6
<211>  11
<212>  PRT
<213>  Artificial Sequence

<220>
<223>  Peptide

<400>  6

Trp Arg Asp Glu Ala Gly Lys Pro Leu Val Lys
1               5                   10

<210>  7
<211>  11
<212>  PRT
<213>  Artificial Sequence

<220>
<223>  Peptide

<400>  7

Trp Lys Asp Glu Ala Gly Lys Pro Leu Met Lys
1               5                   10

<210>  8
<211>  11
<212>  PRT
<213>  Artificial Sequence

<220>
<223>  Peptide

<400>  8

Trp Lys Asp Gln Ala Gly Lys Pro Leu Val Lys
1               5                   10

<210>  9
<211>  11

<212>  PRT
<213>  Artificial Sequence

<220>
<223>  Peptide

<400>  9

Trp Lys Asp Glu Ala Thr Lys Pro Leu Val Lys
1               5                   10


<210>  10
<211>  11
<212>  PRT
<213>  Artificial Sequence

<220>
<223>  Peptide

<400>  10

Tyr Lys Asn Glu Ala Gly Lys Pro Leu Val Lys
1               5                   10


<210>  11
<211>  11
<212>  PRT
<213>  Artificial Sequence

<220>
<223>  Peptide

<400>  11

Trp Lys Asn Glu Ser Gly Lys Pro Leu Val Lys
1               5                   10


<210>  12
<211>  11
<212>  PRT
<213>  Artificial Sequence

<220>
<223>  Peptide

<400>  12

Trp Lys Asp Glu Ala Gly Lys Thr Leu Val Arg
1               5                   10


<210>  13
<211>  11
<212>  PRT
<213>  Artificial Sequence

<220>
<223>  Peptide

<400>  13

Phe Lys Asp Glu Ala Thr Lys Pro Leu Val Lys
1               5                   10

<210>  14
<211>  11
<212>  PRT
<213>  Artificial Sequence

<220>
<223>  Peptide

<400>  14

Phe Lys Asp Glu Ala Gly Lys Pro Leu Ile Lys
1               5                   10

<210>  15
<211>  11
<212>  PRT
<213>  Artificial Sequence

<220>
<223>  Peptide

<400>  15

Trp Lys Asp Glu Ala Gly Lys Thr Leu Leu Lys
1               5                   10

<210>  16
<211>  11
<212>  PRT
<213>  Artificial Sequence

<220>
<223>  Peptide

<400>  16

Trp Lys Asn Glu Ala Gly Lys Pro Val Val Lys
1               5                   10

<210>  17
<211>  11
<212>  PRT
<213>  Artificial Sequence

<220>
<223>  Peptide

<400>  17

Trp Lys Asp Glu Ala Gly Arg Thr Leu Val Lys
1               5                   10

<210>  18
<211>  11

<212>  PRT
<213>  Artificial Sequence

<220>
<223>  Peptide

<400>  18

Trp Glu Asp Glu Ser Gly Lys Pro Leu Leu Lys
1               5                   10


<210>  19
<211>  11
<212>  PRT
<213>  Artificial Sequence

<220>
<223>  Peptide

<400>  19

Trp Lys Glu Glu Ala Gly Lys Pro Ile Val Gln
1               5                   10


<210>  20
<211>  11
<212>  PRT
<213>  Artificial Sequence

<220>
<223>  Peptide

<400>  20

Tyr Lys Asn Glu Ala Gly Lys Pro Leu Val Arg
1               5                   10


<210>  21
<211>  11
<212>  PRT
<213>  Artificial Sequence

<220>
<223>  Peptide

<400>  21

Trp Lys Asp Gln Ala Thr Arg Pro Leu Val Lys
1               5                   10


<210>  22
<211>  11
<212>  PRT
<213>  Artificial Sequence

<220>
<223>  Peptide

<400>  22

Trp Lys Asp Glu Ser Gly Lys Pro Val Leu Lys
1               5                   10

<210>  23
<211>  11
<212>  PRT
<213>  Artificial Sequence

<220>
<223>  Peptide

<400>  23

Trp Gln Asp Asp Ser Gly Lys Pro Leu Val Lys
1               5                   10

<210>  24
<211>  11
<212>  PRT
<213>  Artificial Sequence

<220>
<223>  Peptide

<400>  24

Trp Lys Asn Glu Ala Gly Lys Thr Leu Leu Lys
1               5                   10

<210>  25
<211>  11
<212>  PRT
<213>  Artificial Sequence

<220>
<223>  Peptide

<400>  25

Trp Lys Asp Lys Ala Gly Glu Pro Leu Val Arg
1               5                   10

<210>  26
<211>  11
<212>  PRT
<213>  Artificial Sequence

<220>
<223>  Peptide

<400>  26

Trp Lys Asp Glu Ala Gly Asn Pro Leu Val Lys
1               5                   10

<210>  27
<211>  11

<212>  PRT
<213>  Artificial Sequence

<220>
<223>  Peptide

<400>  27

Cys Lys Asp Glu Ala Gly Lys Pro Leu Val Lys
1               5                   10


<210>  28
<211>  11
<212>  PRT
<213>  Artificial Sequence

<220>
<223>  Peptide

<400>  28

Trp Lys Asp Glu Ala Gly Lys Pro Leu Gly Lys
1               5                   10


<210>  29
<211>  11
<212>  PRT
<213>  Artificial Sequence

<220>
<223>  Peptide

<400>  29

Trp Lys Asp Glu Asn Gly Lys Pro Leu Val Lys
1               5                   10


<210>  30
<211>  11
<212>  PRT
<213>  Artificial Sequence

<220>
<223>  Peptide

<400>  30

Trp Lys Asp Glu Ala Arg Lys Pro Leu Val Lys
1               5                   10


<210>  31
<211>  11
<212>  PRT
<213>  Artificial Sequence

<220>
<223>  Peptide

<400>  31

Trp Lys Asp Glu Ala Gly Lys Pro Leu Val Thr
1               5                   10

<210>  32
<211>  11
<212>  PRT
<213>  Artificial Sequence

<220>
<223>  Peptide

<400>  32

Trp Lys Asp Glu Ala Gly Lys Arg Leu Val Lys
1               5                   10

<210>  33
<211>  11
<212>  PRT
<213>  Artificial Sequence

<220>
<223>  Peptide

<400>  33

Trp Lys Trp Glu Ala Gly Lys Pro Leu Val Lys
1               5                   10

<210>  34
<211>  11
<212>  PRT
<213>  Artificial Sequence

<220>
<223>  Peptide

<400>  34

Trp Lys Asp Glu Ala Gly Phe Pro Thr Val Lys
1               5                   10

<210>  35
<211>  11
<212>  PRT
<213>  Artificial Sequence

<220>
<223>  Peptide

<400>  35

Trp Tyr Asp Met Ala Gly Lys Pro Leu Val Lys
1               5                   10

<210>  36
<211>  11

<212>  PRT
<213>  Artificial Sequence

<220>
<223>  Peptide

<400>  36

Trp Lys Asp Tyr Glu Gly Lys Pro Leu Val Lys
1               5                   10


<210>  37
<211>  11
<212>  PRT
<213>  Artificial Sequence

<220>
<223>  Peptide

<400>  37

Trp Lys Arg Glu Ala Gly Lys Pro Gly Val Lys
1               5                   10


<210>  38
<211>  11
<212>  PRT
<213>  Artificial Sequence

<220>
<223>  Peptide

<400>  38

Trp Lys Leu Glu Lys Gly Lys Pro Leu Val Lys
1               5                   10


<210>  39
<211>  11
<212>  PRT
<213>  Artificial Sequence

<220>
<223>  Peptide

<400>  39

Trp Lys Asp Glu Ala Gly Lys Pro Cys Val Lys
1               5                   10


<210>  40
<211>  11
<212>  PRT
<213>  Artificial Sequence

<220>
<223>  Peptide

<400>  40

Trp Lys Lys Glu Ala Pro Lys Pro Leu Val Lys
1               5                   10

<210>  41
<211>  11
<212>  PRT
<213>  Artificial Sequence

<220>
<223>  Peptide

<400>  41

Ser Lys Asp Glu Ala Gly Pro Pro Leu Val Lys
1               5                   10

<210>  42
<211>  11
<212>  PRT
<213>  Artificial Sequence

<220>
<223>  Peptide

<400>  42

Trp Lys His Glu Pro Gly Lys Pro Leu Ala Lys
1               5                   10

<210>  43
<211>  11
<212>  PRT
<213>  Artificial Sequence

<220>
<223>  Peptide

<400>  43

Trp Lys Asp Glu Arg Glu Lys Pro Phe Val Lys
1               5                   10

<210>  44
<211>  11
<212>  PRT
<213>  Artificial Sequence

<220>
<223>  Peptide

<400>  44

Trp Lys Gln Glu Ala Gly Lys Pro Trp Arg Lys
1               5                   10

<210>  45
<211>  11

<212>  PRT
<213>  Artificial Sequence

<220>
<223>  Peptide

<400>  45

Val Lys Asp Glu Ala Lys Lys Pro Leu Val His
1               5                   10


<210>  46
<211>  11
<212>  PRT
<213>  Artificial Sequence

<220>
<223>  Peptide

<400>  46

Asn Trp Asp Glu Ala Gly Lys Met Leu Val Lys
1               5                   10


<210>  47
<211>  11
<212>  PRT
<213>  Artificial Sequence

<220>
<223>  Peptide

<400>  47

Ile Lys Asp Glu Asp Gly Pro Pro Leu Val Lys
1               5                   10


<210>  48
<211>  11
<212>  PRT
<213>  Artificial Sequence

<220>
<223>  Peptide

<400>  48

Leu Lys Asp Glu Tyr Gly Lys Pro Leu Val Asn
1               5                   10


<210>  49
<211>  11
<212>  PRT
<213>  Artificial Sequence

<220>
<223>  Peptide

<400>  49

Trp Lys Asp Arg Ala Gly Lys Glu Leu Thr Lys
1               5                   10

<210>  50
<211>  12
<212>  PRT
<213>  Artificial Sequence

<220>
<223>  Peptide

<400>  50

Trp Lys Asp Glu Ala Gly Lys Pro Leu Pro Val Lys
1               5                   10

<210>  51
<211>  14
<212>  PRT
<213>  Artificial Sequence

<220>
<223>  Peptide

<400>  51

Trp Lys Gly Asp Glu Asn Tyr Ala Gly Lys Pro Leu Val Lys
1               5                   10

<210>  52
<211>  14
<212>  PRT
<213>  Artificial Sequence

<220>
<223>  Peptide

<400>  52

Leu Trp Lys Asp Glu Ala Gly Arg Lys Tyr Pro Leu Val Lys
1               5                   10

<210>  53
<211>  12
<212>  PRT
<213>  Artificial Sequence

<220>
<223>  Peptide

<400>  53

Trp Lys Asp Cys Glu Gly Ala Gly Lys Pro Leu Val
1               5                   10

<210>  54
<211>  13

<212>  PRT
<213>  Artificial Sequence

<220>
<223>  Peptide

<400>  54

Trp Lys Asp Glu Pro Ala Gly Lys Pro Leu Val Val Lys
1               5                   10


<210>  55
<211>  12
<212>  PRT
<213>  Artificial Sequence

<220>
<223>  Peptide

<400>  55

Trp Lys Asp Glu Ala Gly Pro Lys Pro Leu Val Lys
1               5                   10


<210>  56
<211>  14
<212>  PRT
<213>  Artificial Sequence

<220>
<223>  Peptide

<400>  56

Trp Lys Asp Glu Ala Gly Trp Ala Asp Lys Pro Leu Val Lys
1               5                   10


<210>  57
<211>  12
<212>  PRT
<213>  Artificial Sequence

<220>
<223>  Peptide

<400>  57

Trp Lys Asn Asp Glu Ala Gly Lys Pro Leu Val Lys
1               5                   10


<210>  58
<211>  9
<212>  PRT
<213>  Artificial Sequence

<220>
<223>  Peptide

<400>  58

Trp Lys Asp Ala Lys Pro Leu Val Lys
1               5

<210>  59
<211>  9
<212>  PRT
<213>  Artificial Sequence

<220>
<223>  Peptide

<400>  59

Trp Lys Glu Ala Gly Lys Pro Val Lys
1               5

<210>  60
<211>  10
<212>  PRT
<213>  Artificial Sequence

<220>
<223>  Peptide

<400>  60

Trp Lys Asp Glu Ala Lys Pro Leu Val Lys
1               5                   10

<210>  61
<211>  8
<212>  PRT
<213>  Artificial Sequence

<220>
<223>  Peptide

<400>  61

Trp Asp Glu Ala Gly Lys Pro Val
1               5

<210>  62
<211>  10
<212>  PRT
<213>  Artificial Sequence

<220>
<223>  Peptide

<400>  62

Trp Lys Asp Glu Ala Gly Lys Pro Val Lys
1               5                   10

<210>  63
<211>  9

<212>  PRT
<213>  Artificial Sequence

<220>
<223>  Peptide

<400>  63

Trp Asp Ala Gly Lys Pro Leu Val Lys
1               5


<210>  64
<211>  10
<212>  PRT
<213>  Artificial Sequence

<220>
<223>  Peptide

<400>  64

Trp Lys Asp Glu Ala Gly Lys Pro Leu Val
1               5                   10


<210>  65
<211>  8
<212>  PRT
<213>  Artificial Sequence

<220>
<223>  Peptide

<400>  65

Trp Glu Ala Gly Lys Pro Leu Val
1               5


<210>  66
<211>  6
<212>  PRT
<213>  Artificial Sequence

<220>
<223>  Peptide

<400>  66

Trp Lys Asp Glu Ala Gly
1               5


<210>  67
<211>  5
<212>  PRT
<213>  Artificial Sequence

<220>
<223>  Peptide

<400>  67

Trp Lys Asp Glu Ala
1               5

<210>  68
<211>  8
<212>  PRT
<213>  Artificial Sequence

<220>
<223>  Peptide

<400>  68

Lys Asp Glu Ala Gly Lys Pro Leu
1               5

<210>  69
<211>  5
<212>  PRT
<213>  Artificial Sequence

<220>
<223>  Peptide

<400>  69

Lys Asp Glu Ala Gly
1               5

<210>  70
<211>  7
<212>  PRT
<213>  Artificial Sequence

<220>
<223>  Peptide

<400>  70

Asp Glu Ala Gly Lys Pro Leu
1               5

<210>  71
<211>  5
<212>  PRT
<213>  Artificial Sequence

<220>
<223>  Peptide

<400>  71

Gly Lys Pro Leu Val
1               5

<210>  72
<211>  5

<212>  PRT
<213>  Artificial Sequence

<220>
<223>  Peptide

<400>  72

Asp Glu Ala Gly Lys
1               5


<210>  73
<211>  9
<212>  PRT
<213>  Artificial Sequence

<220>
<223>  Peptide

<400>  73

Trp Lys Asp Glu Ala Gly Lys Pro Leu
1               5


<210>  74

<400>  74
000

<210>  75

<400>  75
000

<210>  76
<211>  5
<212>  PRT
<213>  Artificial Sequence

<220>
<223>  Peptide

<400>  76

Lys Pro Leu Val Lys
1               5


<210>  77
<211>  4
<212>  PRT
<213>  Artificial Sequence

<220>
<223>  Peptide

<400>  77

Trp Lys Asp Glu
1

<210>  78
<211>  5
<212>  PRT
<213>  Artificial Sequence

<220>
<223>  Peptide

<400>  78

Ala Gly Lys Pro Leu
1               5


<210>  79

<400>  79
000

<210>  80

<400>  80
000

<210>  81

<400>  81
000

<210>  82

<400>  82
000

<210>  83

<400>  83
000

<210>  84

<400>  84
000

<210>  85

<400>  85
000

## Description

### Field of the Invention

The present invention relates to cosmetic and pharmaceutical compositions.

### Background to the Invention

Many compositions have been described. Improvement is always desirable.

Currently, there are different approaches for improving muscle health or muscle-glucose-absorption. However, finding an alternative that helps muscle recovery, maintenance and/or muscle growth is desirable.

Indeed, the growing will of maintaining a youthful appearance is leading to more and more research of new cosmetic and dermatological procedures for treatment of skin aging, particularly as people are now living longer and healthier lives. Recently, there has been an increasing enthusiasm for minimally invasive treatments and techniques designed to deal with problems like wrinkles, volume loss and other skin damages. The most common topical anti-ageing solutions are creams and serums.

US2004/0132667 discloses compositions comprising peptides, optionally in combination with one or more additional ingredients. The compositions disclosed provide relief from one or more skin conditions, including those caused by various sources of stress, pollution and general aging.

US2014/0120141 discloses cosmetic and pharmaceutical compositions containing peptides for use in the treatment and/or care of conditions, disorders and/or diseases of the skin and/or mucous membranes.

It is an object of the invention to overcome at least one of the above-referenced problems and provide a composition.

### Summary of the Invention

A first aspect of the invention provides a composition (hereinafter "composition of the invention") comprising an effective amount of a peptide comprising an amino acid sequence of SEQUENCE ID NO. 1 or a variant thereof (hereafter "peptide of the invention").

Preferably, the composition is topical.

Typically, the composition may be a cosmetic composition comprising a cosmetically effective amount of a peptide comprising an amino acid sequence of SEQUENCE ID NO. 1 or a variant thereof.

Suitably, the composition may be a pharmaceutical composition comprising a pharmaceutically effective amount of a peptide comprising an amino acid sequence of SEQUENCE ID NO. 1 or a variant thereof.

Preferably, said peptide is a bioactive peptide.

Typically, said variant is a bioactive variant.

Suitably, the composition comprises a plurality of peptides.

Typically, the composition further comprises at least one cosmetically or pharmaceutically acceptable excipient or additive.

Typically, the composition further comprises at least one cosmetically or pharmaceutically acceptable active.

Suitably, the peptide comprises an amino acid sequence consisting of SEQUENCE ID NO. 1. Typically, said variant has from about 70% to about 99% sequence identity with SEQUENCE ID NO. 1.

Typically, said variant has at least about 70%, 75%, 80%, 85%, 90% or 95% sequence identity with SEQUENCE ID NO. 1.

Preferably, said variant has an amino acid sequence comprising any one of SEQUENCE ID NO. 2 to SEQUENCE ID NO. 85.

Typically, said variant has an amino acid sequence consisting of any one of SEQUENCE ID NO. 2 to SEQUENCE ID NO. 85.

Typically, said peptide comprises from about 3 to 50 amino acids in length. Preferably from about 5 to about 20 amino acids in length, preferably about 11 amino acids in length.

Typically, said variant comprises from about 3 to about 50 amino acids in length. Preferably from about 5 to about 20 amino acids in length, preferably about 11 amino acids in length.

Typically, said variant is a fragment of SEQ ID NO. 1 comprising at least three amino acids in length. Typically, said fragment is bioactive.

Preferably, said variant is a fragment of SEQ ID NO. 1 having at least 4, 5, 6, 7, 8, 9, 10, or 11, amino acids in length.

Preferably, said peptide or variant has an activity selected from one or more of anti-aging activity, glucose transport-promoting activity, cellular growth promoting activity or anabolic activity. The activity may be cosmetic, i.e. non-therapeutic, therapeutic or both.

Suitably, the peptide or variant has anti-ageing activity.

Suitably, the peptide or variant has glucose transport-promoting activity.

Suitably, the peptide or variant has cellular growth promoting activity.

Suitably, the peptide or variant has anabolic activity. Typically, the anabolic activity is muscle growth. The peptide or variant stimulates anabolic metabolism in a mammal.

Still preferred, the peptide comprises (or consists of) an amino acid sequence of SEQUENCE ID NO. 1, or the variant comprises (or consists of) an amino acid sequence of any one of SEQUENCE ID NO.2 to 85, wherein the peptide or variant typically has anti-aging activity.

Still preferred, the peptide comprises (or consists of) an amino acid sequence of SEQUENCE ID NO. 1, or the variant comprises (or consists of) an amino acid sequence of any one of SEQUENCE ID NO.2 to 85, wherein the peptide or variant typically has glucose transport-promoting activity.

Still preferred, the peptide comprises (or consists of) an amino acid sequence of SEQUENCE ID NO 1, or the variant comprises (or consists of) an amino acid sequence of any one of SEQUENCE ID NO.2 to 85, wherein the peptide or variant typically cellular growth promoting activity.

Still preferred, the peptide comprises (or consists of) an amino acid sequence of SEQUENCE ID NO. 1, or the variant comprises (or consists of) an amino acid sequence of any one of SEQUENCE ID NO.2 to 85, wherein the peptide or variant typically has anabolic activity.

A further aspect of the current invention provides a non-therapeutic use of the composition of the invention as a cosmetic.

A further aspect of the current invention provides a composition of the invention as a medicament.

The following aspects may be cosmetic, i.e. non-therapeutic, or therapeutic.

An aspect of the current invention provides a composition for use a method for anti-ageing.

The invention also provides a composition of the invention for use in a method for slowing or inhibiting, or preventing ageing of human skin. Typically, administration may be by means of a plaster or patch or a formulation suitable for topical application.

A further aspect of the invention provides a composition of the invention for use in a method of promoting growth of tissue.

The invention also provides a composition of the invention for use in a method for promoting growth of epithelial tissue.

The invention also provides a composition of the invention for use in a method for promoting growth of skin.

The invention also provides a composition of the invention for use in a method for promoting growth of an organ.

The invention also provides a composition of the invention for use in a method for promoting growth of an organism.

Preferably, the cell, tissue or organism has a normal pathology (for example ageing skin). Typically, the cell, tissue or skin has abnormal pathology (for example tissue damaged due to trauma, drug use, or epithelial tissue in the GI tract damaged due to an inflammatory disorder).

The growth promoting uses may be *in-vivo* or *in-vitro* uses. The growth promoting uses may involve administration to mammal externally (*i.e.* to the skin) or internally (*i.e.* to the GI tract).

A further aspect of the current invention provides a composition of the invention for use in a method for improving muscle status in a mammal.

A further aspect of the current invention provides a composition of the invention for use in a method for promoting recovery of muscle, typically following exercise.

A further aspect of the current invention provides a composition of the invention for use in method for maintaining or restoring muscle health (for example lean tissue mass) in a mammal.

A further aspect of the current invention provides a composition of the invention for use in a method for enhancing physical performance by topical administration.

A further aspect of the current invention provides a composition of the invention for use in a method for the treatment or prevention of a disease or condition characterised by lethargy or low energy levels.

A further aspect of the current invention provides a composition of the invention for use in a method for muscle growth or muscle building. The composition of the invention may be used in a method for increasing lean muscle mass in beef cattle and other farm animals.

A further aspect of the current invention provides a composition of the invention for use in a method for increasing protein synthesis.

A further aspect of the current invention provides a composition of the invention for use in a method for the treatment of muscle loss.

Another aspect of the invention provides the composition of the invention for use in a method for the treatment of a wound in a mammal.

A still further aspect of the invention provides a composition of the invention for use in a method for the treatment or prevention of pain in a mammal.

Another aspect of the invention provides the composition for use in a method for the treatment or prevention of a disease or condition characterised by damaged epithelial cells or tissue, and/or damaged dermal or epithelial cells or tissue. Preferably, the disease or condition is characterised by damaged dermal or epithelial cells or tissue is selected from cancer, trauma

A further aspect of the invention relates to a method of treating, preventing or caring for any one of the aforementioned and described herein diseases, conditions and/or disorders comprising a step of administering the composition of the invention. The composition may be administered topically.

The uses of the invention may be therapeutic or cosmetic, i.e. non-therapeutic.

A further aspect of the invention relates to a composition comprising one or more of the peptides selected from SEQUENCE ID NO. 1 to 85.

In one embodiment, the composition of the invention comprises substantially all of the peptides of SEQUENCE ID NO. 1 to 85.

A further aspect of the current invention relates to a man-made treatment composition comprising the composition of the invention. Preferably, the treatment composition is an anti-aging composition. Preferably, the treatment is a muscle treatment composition. In an embodiment, the composition is topical. In one embodiment, the composition comprises a cream, gel, lotion, rub, powder. Preferably, the treatment is a wound treatment composition.

The invention also relates to a plaster, bandage or dressing suitable for application to the keratinous tissue or a wound and comprising the composition of the invention.

Preferably the composition or product is man-made.

### Definitions

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an effective amount or a therapeutically effective amount of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In this specification, the term "composition" should be understood to mean something made by the hand of man, and not excludes naturally occurring compositions.

The term "bioactive" when used herein refers to a peptide or a peptide variant that has biological activity. For example, the biological activity may be one or more of glucose transport promoting activity, growth promoting activity, anti-ageing activity, anabolic activity, GLUT4 translocation promotion activity and protein synthesis promotion activity. The activity may be anti-inflammatory activity or anti-bacterial activity. The activity may be antioxidation.

"Glucose transport promoting" or "glucose transport promoting activity" as applied to a peptide or variant or fragment means a peptide, variant or fragment that is capable of increasing cellular glucose uptake in the glucose uptake assay described below.

"GLUT4 translocation promoting" or "GLUT4 translocation promotion activity" as applied to a peptide, variant or fragment means a peptide, variant or fragment that is capable of increasing GLUT4 translocation into skeletal muscle compared with an untreated control in the *in vitro* assay described below.

"Anti-ageing" means inhibiting or slowing the appearance of ageing of a human's skin and/or reversing the appearance of ageing. "Slowing or inhibiting ageing of the skin" means slowing or inhibiting the ageing process in the skin, and/or reversing the appearance of ageing. In one embodiment, "anti-ageing" or "anti-ageing activity" as applied to a peptide or variant or fragment means one that is capable of increasing collagen production or elastin production in Human Dermal Fibroblasts compared with an untreated control when tested in an *in-vitro* assay as described below and/or capable of increasing cell proliferation in the cell proliferation assay described below.

"Cellular growth promoting" as applied to a peptide, variant or fragment means a peptide, variant or fragment that is capable of increasing elastin production or collagen production or cellular proliferation in an assay as described below. Peptides or peptide variants which increase cell proliferation or have "cellular growth promoting activity" are anti-ageing peptides or variants.

"Increasing protein synthesis" as applied to a peptide of the invention means a peptide that is capable of significantly increasing the degree of phosphorylation of MTOR in the Phospho-MTOR assay described below or significantly increasing the degree of protein synthesis in the puromycin assay described below. Peptides and compositions that increase protein synthesis may be employed to stimulate anabolic metabolism in a mammal.

"Anti-inflammatory" as applied to a peptide, variant or fragment means a peptide, variant or fragment that is capable of significantly reducing the secretion of TNFα by LPS-stimulated J774.2 macrophages (compared with untreated LPS-stimulated J774.2 macrophages) when the macrophages are treated with 100µM of the peptide, variant or fragment. J774.2 macrophages were treated with 100µM of synthetic peptide for 24 hours and then stimulated with (A) LPS (10ng/ml) for five hours or (B) LPS (10ng/ml) for 5 hours followed by ATP (5mM) for one hour. Supernatant was collected and levels of TNFα were determined by ELISA.

"Antibacterial" or "antibacterial activity" as applied to a peptide, variant or fragment means a peptide, variant or fragment that is capable of visibly inhibiting the growth of a bacteria in the following agar-plate based growth inhibition assay: Peptide stock=5mg/mL dissolved in DMSO. Bacterial inoculums were adjusted to McFarland 0.5 standard and MHA plates swabbed. Blank disks were placed in the plates and 10 µL of each compound (at 64 µg/mL-maximum concentration tested) added. Plates were incubated at 37°C for 16-18 hours. Appropriate controls (DMSO; Mueller-Hinton media alone; and two antibiotic discs - ciprofloxacin and tetracycline) were also performed.

The term "topical composition" refers to a composition that is formulation for topical administration. "Topical administration" refers to the application to the keratinous tissue, such as the skin, hair and nails. Topical delivery generally means delivery to the skin, but can also mean delivery to a body lumen lined with epithelial cells, for example the lungs or airways, the gastrointestinal tract, the buccal cavity. In particular, formulations for topical delivery are described in Topical drug delivery formulations edited by David Osborne and Antonio Aman, Taylor & Francis, the complete contents of which are incorporated herein by reference. Compositions or formulations for delivery to the airways are described in O'Riordan et al (Respir Care, 2002, Nov. 47), EP2050437, WO2005023290, US2010098660, and US20070053845. Composition and formulations for delivering active agents to the iluem, especially the proximal iluem, include microparticles and microencapsulates where the active agent is encapsulated within a protecting matrix formed of polymer or dairy protein that is acid resistant but prone to dissolution in the more alkaline environment of the ileum. Examples of such delivery systems are described in EP1072600.2 and EP13171757.1. An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required. Injectable forms may contain between 10-1000 mg, preferably between 10-250 mg, of active ingredient per dose.

Compositions may be formulated in unit dosage form, i.e., in the form of discrete portions containing a unit dose, or a multiple or sub-unit of a unit dose.

The term "cosmetic composition" when used herein relates to a composition that can be used for cosmetic purposes, personal care and/or hygiene purposes. It will be appreciated that the composition may have more than one cosmetic purpose and may be used for more than one of these purposes at the same time. A "cosmetic" when used herein can include but are not limited to, lipstick, mascara, rouge, foundation, blush, eyeliner, facial and body powder, sunscreen, sunblock, nail polish, compacts, solids, pencils.

"Pharmaceutical compositions": A further aspect of the invention relates to a pharmaceutical composition comprising a peptide of the invention or a composition of peptides of the invention, admixed with one or more pharmaceutically acceptable diluents, excipients or carriers. Even though the peptides and compositions of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients, 2nd Edition, (1994), Edited by A Wade and PJ Weller. In particular, formulations for topical delivery are described in Topical drug delivery formulations edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985). Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of phydroxybenzoic acid. Antioxidants and suspending agents may be also used.

The term "mammal" should be understood to mean a higher mammal, especially a human. However, the term also includes non-mammalian animals such as fish. The human may be an infant, toddler, child, adolescent, adult, or elderly human. In one embodiment of the invention, the human is an elderly person, for example aged 55 or more. In one embodiment, the human is an elderly person experiencing deterioration of lean tissue mass. In one embodiment, the human is a sportsperson. In one embodiment, the human is pregnant woman. In one embodiment, the human is suffering from lethargy or perceived lack of energy.

The term "dermatologically acceptable," as used herein, means that the topical composition(s) or component(s) of the composition(s) are suitable for use in contact with human skin or keratinous tissue without risk of toxicity, incompatibility, instability and/or allergic response, and similar.

The term "sustained release" is used in a conventional sense relating to a delivery system of a compound or active, which provides the gradual release of this compound or active during a period of time and preferably, although not necessarily, with relatively constant compound release levels over a period of time.

The term "peptide" used herein refers to a polymer composed of up to 50 amino acids, for example 5 to 50 amino acid monomers typically linked via peptide bond linkage. Peptides (including fragments and variants thereof) of and for use in the invention may be generated wholly or partly by chemical synthesis or by expression from nucleic acid. For example, the peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984). When necessary, any of the peptides employed in the invention can be chemically modified to increase their stability. A chemically modified peptide or a peptide analog includes any functional chemical equivalent of the peptide characterized by its increased stability and/or efficacy in vivo or in vitro in respect of the practice of the invention. The term peptide analog also refers to any amino acid derivative of a peptide as described herein. A peptide analog can be produced by procedures that include, but are not limited to, modifications to side chains, incorporation of unnatural amino acids and/or their derivatives during peptide synthesis and the use of cross-linkers and other methods that impose conformational constraint on the peptides or their analogs. Examples of side chain modifications include modification of amino groups, such as by reductive alkylation by reaction with an aldehyde followed by reduction with NaBH₄; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH₄. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. Peptide structure modification includes the generation of retro-inverso peptides comprising the reversed sequence encoded by D-amino acids. Changes may be those that reduce susceptibility to proteolysis, reduce susceptibility to oxidation, alter binding affinity of the variant sequence (typically desirably increasing affinity), and/or confer or modify other physicochemical or functional properties on the associated variant/analog peptide

A "variant" of the peptide shall be taken to mean a peptide having an amino acid sequence that is substantially identical to SEQUENCE ID NO.1, but which is altered in respect of one or more amino acid residues. Preferably such alterations involve the insertion, addition, deletion and/or substitution of 11 or fewer amino acids, more preferably of 10 or fewer, 9 or fewer, 8 or fewer, 7 or fewer, 6 or fewer, preferably 5 or fewer, 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Generally, the variant will have at least 70% amino acid sequence identity, preferably at least 80% sequence identity, more preferably at least 90% sequence identity, and ideally at least 95%, 96%, 97%, 98% or 99% sequence identity with the parent sequence.

The term variant is also taken to encompass the term "fragment" and as such means a segment of amino acid SEQUENCE ID NO. 1. Typically, the fragment has between 3 and 13 contiguous amino acids in length. Generally, the fragment has a charge of -5 to +3. The charge of a peptide, fragment or region is determined using the method of Cameselle, J.C., Ribeiro, J.M., and Sillero, A. (1986). Derivation and use of a formula to calculate the net charge of acid-base compounds. Its application to amino acids, proteins and nucleotides. Biochem. Educ. 14, 131-136.

In this specification, the term "sequence identity" should be understand to comprise both sequence identity and similarity, *i.e.* a variant (or homolog) that shares 70% sequence identity with a reference sequence is one in which any 70% of aligned residues of the variant (or homolog) are identical to, or conservative substitutions of, the corresponding residues in the reference sequence across the entire length of the sequence. Sequence identity is the amount of characters which match exactly between two different sequences. Hereby, gaps are not counted and the measurement is relational to the shorter of the two sequences.

In terms of "sequence homology", the term should be understood to mean that a variant (or homolog) which shares a defined percent similarity or identity with a reference sequence when the percentage of aligned residues of the variant (or homolog) are either identical to, or conservative substitutions of, the corresponding residues in the reference sequence and where the variant (or homolog) shares the same function as the reference sequence.

This alignment and the percent homology or sequence identity can be determined using software programs known in the art, for example, one alignment program is BLAST, using default parameters. Details of these programs can be found at the following Internet address: http://www.ncbi.nlm.nih.gov/blast/Blast.cgi.

"Inflammatory disorder" means an immune-mediated inflammatory condition that affects humans and is generally characterised by dysregulated expression of one or more cytokines. Examples of inflammatory disorders include skin inflammatory disorders and inflammatory disorders of the joints, Examples of skin inflammatory disorders include dermatitis, for example atopic dermatitis and contact dermatitis, acne vulgaris, and psoriasis. Examples of inflammatory disorders of the joints include rheumatoid arthritis. The peptides and compositions of the invention may also be employed in the non-therapeutic treatment of inflammation. Examples of non-therapeutic treatment of inflammation include use to relieve normal, non-pathological, inflammation, for example inflammation in the muscles and joints following exercise.

"Disease or condition characterised by damaged dermal or epithelial cells or tissue" means any condition or disease that results in damaged dermal or epithelial tissue or cells or organs. One example is trauma which often results in damaged skin. Another example is an inflammatory skin condition such as psoriasis or eczema which often results in damaged skin. Another example is an inflammatory disorder of the lower intestines which can result in damaged epithelial cells/tissue lining the lower intestines. Another example is damaged epithelial cells/tissue lining the lower intestines caused by ingestion of a toxic or damaging substance, for example toxic chemicals or drugs. Another example is cancer, for example bowel cancer, which can result in damaged epithelial tissue in the bowel. Another condition is a peripheral inflammatory disorder such as atopic dermatitis which can result in damage to the skin in humans.

"Disease or condition characterised by bacterial infection" means any condition or disease characterised having a pathology caused by growth of bacteria or by bacterial infection, including for example MRSA, salmonella, listeria. Specific examples are provided in https://en.wikipedia.org/wiki/List of infectious diseases.

"Man-made" as applied to comestible products should be understood to mean made by a human being and not existing in nature.

"Improving muscle status" means improving the muscle health, for example promoting skeletal muscle protein synthesis, skeletal glucose absorption, improving lean tissue mass in therapeutic or non-therapeutic context, promoting muscle recovery generally after activity exercise, or improving muscle performance. The methods or uses may be therapeutic or non-therapeutic. The term "improving lean tissue mass status" should be understood to mean increasing lean tissue mass, or inhibiting or preventing the rate of lean tissue mass degradation.

"Promoting muscle recovery" means causing an increase in absorption of glucose in skeletal muscle compared with untreated skeletal muscle.

"Maintaining or restoring muscle health" means helping retain or restore mammalian muscle health resulting from damage incurred during exercise. By promoting glucose transport in skeletal muscle the peptides promote recovery from exercise, and relieve muscle soreness/pain and injury connected with exercise. They can also be used to decrease and prevent muscle cramping, and to allow a faster recovery from muscle cramping. Cramping can result from physical stress, mental stress, and or Repetitive Strain Injury stress. By promoting glucose transport the peptides help reduce Myopathy of the muscle, and help prevent Sarcopenia in mammals, promote recovery from injuries during exercise, and relieve muscle soreness/pain and injury connected with exercise. The invention also relates to a peptide or composition of the invention for use in maintaining or restoring muscle health in a mammal.

In this specification, the term "personal care product" should be understood to mean a composition formulated for use by humans in cleaning or treating the human body, particularly the skin, teeth, nails, feet and hair. Examples include shampoo, conditioner, skin creams and lotions, powders, dentifrice, shower gel or creams, bath or shower gel, hair dye, soap, body scrub, exfoliant, anti-dandruff solutions body lotion, shaving solutions, moisturisers, cleaners, masks, oils, serums, and rinses, deodorant, and anti-perspirant.

The term "skin aging" is used in the sense in which it is generally and widely used in the art of cosmetic and personal care products. Signs of skin aging include wrinkles, lines, crevices, bumps, red spots, large pores, roughness, dullness, loss of elasticity, sagging, loss of tightness, discoloration, blotching, hyperpigmentation, freckles, keratosis, inflammation, collagen breakdown and other histological changes in the skin layers including underlying tissue.
The term "cosmetically or pharmaceutically acceptable salts" means a salt recognized for its use in animals and more specifically in human beings, and includes salts used to form base addition salts, either they are inorganic, such as and not restricted to, lithium, sodium, potassium, calcium, magnesium, manganese, copper, zinc or aluminium among others, either they are organic, such as and not restricted to, ethylamine, diethylamine, ethylenediamine, ethanolamine, diethanolamine, arginine, lysine, histidine or piperazine among others, or acid addition salts, either they are organic, such as and not restricted to, acetate, citrate, lactate, malonate, maleate, tartrate, fumarate, benzoate, aspartate, glutamate, succinate, oleate, trifluoroacetate, oxalate, pamoate or gluconate among others, or inorganic, such as and not restricted to, chloride, sulfate, borate or carbonate, among others. The nature of the salt is not critical, provided that it is cosmetically or pharmaceutically acceptable. The cosmetically or pharmaceutically acceptable salts of the peptides of the invention can be obtained by the conventional methods, well known in the prior art [Berge S. M. et al., "Pharmaceutical Salts", J. Pharm. Sci., (1977), 66, 1-19].

"C-terminal domain" as applied to a fragment means the first three amino acids at the c-terminus of the fragment.

"N-terminal domain" as applied to a fragment means the last three amino acids at the n-terminus of the fragment.

"Homolog" of a reference protein should be understood to mean a protein from a different species of plant having at least 60%, 70%, 80%, 90%, 91%, 92%, 93%, 94%, 95%, 96%, 97%, 98%, 99% sequence homology with the reference protein.

In the specification, the terms "comprise, comprises, comprised and comprising" or any variation thereof and the terms "include, includes, included and including" or any variation thereof are considered to be totally interchangeable and they should all be afforded the widest possible interpretation and vice versa.

### Brief Description of the Drawings

The invention will be more clearly understood from the following description of an embodiment thereof, given by way of example only, with reference to the accompanying drawings, in which: -
**Figure 1** illustrates results of a cell proliferation assay using untreated and peptide-treated Human Dermal Fibroblasts (HDF).
**Figure 2** illustrates the results of a cell proliferation assay using untreated and peptide-treated HACAT cells.
**Figure 3** illustrates the results of a collagen expression assay using untreated and peptide-treated Human Dermal Fibroblasts (HDF).
**Figure 4** illustrates the results of an elastin expression assay using untreated and peptide-treated Human Dermal Fibroblasts (HDF).
**Figure 5** illustrates the results of a glucose uptake assay using untreated and peptide-treated Human skeletal myoblasts.
**Figure 6** illustrates the results of a glucose uptake assay using untreated and peptide-treated Human skeletal myoblasts.
**Figure 7** illustrates the results of a glucose uptake assay using untreated and peptide-treated Human skeletal myoblasts.
**Figure 8** illustrates the results of a GLUT4 translocation assay assay using untreated and peptide-treated Human skeletal myoblasts.
**Figure 9** illustrates the results of a muscle protein synthesis (mTOR) assay.
**Figure 10** illustrates the results of a muscle protein synthesis (puromycin) assay.

### Detailed Description of the Drawings

In the broadest sense, the first aspect of the invention provides a composition comprising an effective amount of a peptide comprising an amino acid sequence of SEQUENCE ID NO. 1 or a variant thereof (hereafter "peptide of the invention").

The composition may be topical.

The composition may be a cosmetic composition comprising a cosmetically effective amount of a peptide comprising an amino acid sequence of SEQUENCE ID NO. 1 or a variant thereof.

The composition may be a pharmaceutical composition comprising a pharmaceutically effective amount of a peptide comprising an amino acid sequence of SEQUENCE ID NO. 1 or a variant thereof.

It will be understood that the composition may have a cosmetic effect, *i.e.* non-therapeutic or a therapeutic effect. The composition may have a cosmetic and a therapeutic effect.

Examples of cosmetic applications include, but are not limited to, anti-ageing, slowing or inhibiting, or preventing ageing of human skin, promoting growth of tissue, promoting growth of epithelial tissue, promoting growth of skin, promoting growth of an organ, promoting growth of an organism, improving muscle status in a mammal, promoting recovery of muscle, typically following exercise, maintaining or restoring muscle health in a mammal, enhancing physical performance, muscle growth or muscle building and treatment of muscle loss.

Examples of therapeutic applications include, but are not limited to, promoting growth of tissue, promoting growth of epithelial tissue, promoting growth of skin, promoting growth of an organ, promoting growth of an organism, improving muscle status in a mammal, promoting recovery of muscle, maintaining or restoring muscle health in a mammal, muscle growth or muscle building, treatment of muscle loss, treatment or prevention of a disease or condition characterised by lethargy or low energy levels, treatment of a wound in a mammal, treatment or prevention of pain in a mammal, treatment or prevention of a disease or condition characterised by damaged epithelial cells or tissue, and/or damaged dermal or epithelial cells or tissue.

SEQUENCE ID NO.1 has the following sequence:
WKDEAGKPLVK

In an embodiment of the invention, the peptide or variant is bioactive.

In an embodiment, the peptide or variant has glucose transport promoting activity. In one embodiment, the peptide or variant has cellular growth promoting activity. In one embodiment, the peptide or variant has anti-aging activity. In an embodiment, the peptide or variant has anabolic activity. It will be appreciated that the peptide or variant may have two or three of glucose transport promoting activity, cellular growth promoting activity, anti-aging activity and anabolic activity. The peptide or variant may have glucose transport promoting activity, cellular growth promoting activity, anti-aging activity and anabolic activity.

In an embodiment of the invention the peptide comprises from about 3 to 50 amino acids in length, about 14 to about 50 amino acids in length, preferably about, 15, 20, 25, 30, 35, 40, 45, or 49 amino acids in length, preferably about 14, 15, 16, 17, 18, 19, or 20 amino acids in length.

In an embodiment of the invention the variant comprises from about 3 to about 11 amino acids in length, preferably about, 4, 5, 6, 7, 8, 9, or 10, amino acids in length.

In one embodiment, the variant has 1 to 5 amino acid changes compared to SEQUENCE ID NO: 1. In one embodiment, the variant has 1 to 4 amino acid changes compared to SEQUENCE ID NO: 1. In one embodiment, the variant has 1 to 3 amino acid changes compared to SEQUENCE ID NO: 1. In one embodiment, the variant has 1 to 2 amino acid changes compared to SEQUENCE ID NO: 1. In one embodiment, the amino acid change is an amino acid substitution. In one embodiment, the amino acid substitution is a conservative substitution. In one embodiment, the amino acid change is an amino acid addition. In one embodiment, the amino acid change is an amino acid deletion.

### The variants of the invention include: -

### Variants of SEQUENCE ID NO: 1 (Pep_260)

Variants of SEQUENCE ID NO: 1 (WKDEAGKPLVK) including variants having 1,2 or 3 conservative amino acid substitutions, 1, 2 to 3 non-conservative amino acid substitutions, 1, 2 or 3 amino acid additions, 1, 2 or 3 amino acid deletions, are provided below:
One conservative amino acid substitution:
   WKEEAGKPLVK (SEQ ID NO.2); FKDEAGKPLVK (SEQ ID NO.3); WKDEAGKPMVK (SEQ ID NO.4); WKDEAGRPLVK (SEQ ID NO.5); WRDEAGKPLVK (SEQ ID NO.6); WKDEAGKPLMK (SEQ ID NO.7); WKDQAGKPLVK (SEQ ID NO.8); WKDEATKPLVK (SEQ ID NO.9)
Two conservative amino acid substitutions:
   YKNEAGKPLVK (SEQ ID NO.10); WKNESGKPLVK (SEQ ID NO.11); WKDEAGKTLVR (SEQ ID NO.12); FKDEATKPLVK (SEQ ID NO. 13); FKDEAGKPLIK (SEQ ID NO. 14); WKDEAGKTLLK (SEQ ID NO. 15); WKNEAGKPVVK (SEQ ID NO. 16); WKDEAGRTLVK (SEQ ID NO. 17)
Three conservative amino acid substitutions:
   WEDESGKPLLK (SEQ ID NO.18); WKEEAGKPIVQ (SEQ ID NO.19); YKNEAGKPLVR (SEQ ID NO.20); WKDQATRPLVK (SEQ ID NO. 21); WKDESGKPVLK (SEQ ID NO.22); WQDDSGKPLVK (SEQ ID NO.23); WKNEAGKTLLK (SEQ ID NO.24); WKDKAGEPLVR (SEQ ID NO.25)
One non-conservative amino acid substitution:
   WKDEAGNPLVK (SEQ ID NO. 26); CKDEAGKPLVK (SEQ ID NO.27); WKDEAGKPLGK (SEQ ID NO.28); WKDENGKPLVK (SEQ ID NO.29); WKDEARKPLVK (SEQ ID NO.30); WKDEAGKPLVT (SEQ ID NO.31); WKDEAGKRLVK (SEQ ID NO.32); WKWEAGKPLVK (SEQ ID NO.33)
Two non-conservative amino acid substitution:
   WKDEAGFPTVK (SEQ ID NO. 34); WYDMAGKPLVK (SEQ ID NO. 35); WKDYEGKPLVK (SEQ ID NO. 36); WKREAGKPGVK (SEQ ID NO. 37); WKLEKGKPLVK (SEQ ID NO. 38); WKDEAGKPCVK (SEQ ID NO. 39); WKKEAPKPLVK (SEQ ID NO. 40); SKDEAGPPLVK (SEQ ID NO. 41)
Three non-conservative amino acid substitution:
   WKHEPGKPLAK (SEQ ID NO. 42); WKDEREKPFVK (SEQ ID NO. 43); WKQEAGKPWRK (SEQ ID NO. 44); VKDEAKKPLVH (SEQ ID NO. 45); NWDEAGKMLVK (SEQ ID NO. 46); IKDEDGPPLVK (SEQ ID NO. 47); LKDEYGKPLVN (SEQ ID NO. 48); WKDRAGKELTK (SEQ ID NO. 49)
Amino acid additions
   WKDEAGKPLPVK (SEQ ID NO. 50); WKGDENYAGKPLVK (SEQ ID NO.51); LWKDEAGRKYPLVK (SEQ ID NO.52); WKDCEGAGKPLVK (SEQ ID NO.53); WKDEPAGKPLVVK (SEQ ID NO.54); WKDEAGPKPLVK (SEQ ID NO.55); WKDEAGWADKPLVK (SEQ ID NO.56); WKNDEAGKPLVK (SEQ ID NO.57)
Amino acid deletions
   WKDAKPLVK (SEQ ID NO. 58); WKEAGKPVK (SEQ ID NO. 59); WKDEAKPLVK (SEQ ID NO. 60); WDEAGKPV (SEQ ID NO. 61); WKDEAGKPVK (SEQ ID NO. 62); WDAGKPLVK (SEQ ID NO. 63); WKDEAGKPLV (SEQ ID NO. 64); WEAGKPLV (SEQ ID NO. 65)

The variant may be a bioactive variant.

In one embodiment, the variant is an anti-aging variant. In one embodiment, the variant has cellular growth promoting activity. In one embodiment, the variant is a glucose transport promoting variant. In one embodiment, the variant is an anabolic variant. It will be appreciated that in one embodiment the bioactive variant is two or more of an anti-aging variant, glucose transport promoting variant, cellular growth promoting variant and an anabolic variant. In one embodiment, the bioactive variant is an anti-aging variant, glucose transport promoting variant, cellular growth promoting variant and an anabolic variant.

The invention also provides fragments of SEQ ID NO. 1, and peptides comprising one or more of these fragments.

The fragment may be a bioactive fragment. In one embodiment, the fragment is an anti-aging fragment. In one embodiment, the fragment is a cellular growth promoting fragment. In one embodiment, the fragment is a glucose transport promoting fragment. In one embodiment, the fragment is an anabolic fragment It will be appreciated that in one embodiment the bioactive fragment is two or more of an anti-aging fragment, glucose transport promoting fragment, cellular growth promoting fragment and an anabolic fragment. In one embodiment, the bioactive fragment is an anti-aging fragment, glucose transport promoting fragment, cellular growth promoting fragment and an anabolic fragment.

Examples of fragments of SEQ ID NO: 1 are provided below:
WKDEAG (SEQ ID NO. 66); WKDEA (SEQ ID NO. 67); KDEAGKPL (SEQ ID NO. 68); KDEAG (SEQ ID NO. 69); DEAGKPL (SEQ ID NO. 70); GKPLV (SEQ ID NO. 71); DEAGK (SEQ ID NO. 72); WKDEAGKPL (SEQ ID NO. 73); WKD (SEQ ID NO. 74); KDE (SEQ ID NO. 75); KPLVK (SEQ ID NO. 76); WKDE (SEQ ID NO. 77); AGKPL (SEQ ID NO. 78); EAG (SEQ ID NO. 79); AGK (SEQ ID NO. 80); KPL (SEQ ID NO. 81); LVK (SEQ ID NO. 82); GKP (SEQ ID NO. 83); DEA (SEQ ID NO. 84); PLV (SEQ ID NO. 85)

It will be appreciated that the composition may comprise a plurality of peptides, fragments and/or variants. Preferably, the composition comprises at least two peptides of the invention. Preferably, the composition comprises at least three peptides of the invention. Preferably, the composition comprises at least four peptides of the invention. Preferably, the composition comprises at least five peptides of the invention. Preferably, the composition comprises at least six peptides of the invention. Preferably, the composition comprises at least seven peptides of the invention. Preferably, the composition comprises at least eight peptides of the invention. Preferably, the composition comprises at least nine peptides of the invention. Preferably, the composition comprises at least ten peptides of the invention. In one embodiment, the composition comprises substantially all the peptides. In one embodiment, the composition comprises substantially all the variants. In one embodiment, the composition is substantially free of other peptides.

In an embodiment, the peptide, variant or composition of the invention has an activity selected from one or more of anti-bacterial activity, anti-inflammatory activity and anti-oxidant activity. The activity may be cosmetic, *i.e.* non-therapeutic, therapeutic or both. The invention also provides a composition of the invention for use in a method of maintaining or restoring gut health in a mammal. The invention also provides a composition of the invention for use in a method for the treatment of a bacterial infection. A further aspect of the invention provides a composition of the invention for use in a method for the treatment or prevention of an inflammatory disorder and/or inflammation in a mammal. Preferably, the inflammation is symptomatic inflammation. This use may be in addition to the above discussed uses of the invention or as an alternative.

The composition may be a topical composition. The topical composition may be presented in a formulation selected from the group comprising creams, multiple emulsions, anhydrous compositions, aqueous dispersions, oils, milks, balsams, foams, lotions, gels, cream gels, hydro-alcoholic solutions, hydro-glycolic solutions, cosmetic, personal care product, hydrogels, liniments, sera, soaps, dusting powder, paste, semi solid formulations, liniments, serums, shampoo, conditioner, ointments, any rinse off formulation, talc, mousses, powders, sprays, aerosols, solutions, suspensions, emulsions, syrups, elixirs, polysaccharide films, patches, gel patches, bandages, an adhesive system, water-in-oil emulsions, oil-in-water emulsions, and silicone emulsions.

In an embodiment of the current invention, the emulsion contains a lipid or oil. The emulsion may be, but is not limited to, oil-in-water, water-in-oil, water-in-oil-in-water and oil-in-water-in-silicone emulsions. The emulsion may contain a humectant. The emulsion may contain an anti-foaming agent, such as silicone. The emulsion may have any suitable viscosity. Emulsions may further contain an emulsifier and/or an anti-foaming agent. Methods of preparing an emulsion are known to a person skilled in the art.

The composition of the invention may be presented, prepared and/or administered in a variety of suitable forms. Such forms include, for example, but are not limited to, liquid, semi-solid and solid dosage forms, such as liquid solutions (e.g., injectable and infusible solutions), dispersions or suspensions, emulsions, microemulsions, tablets, pills, powders, liposomes, dendrimers and other nanoparticles, microparticles, and suppositories. It will be appreciated that the form may depend on the intended mode of administration, the nature of the composition or combination, and therapeutic application or other intended use. Formulations also can include, for example, powders, pastes, ointments, jellies, waxes, oils, lipids, lipid (cationic or anionic) containing vesicles, DNA conjugates, anhydrous absorption pastes, oil-in-water and water-in-oil emulsions, emulsions, carbowax (polyethylene glycols of various molecular weights), semi-solid gels, and semi-solid mixtures containing carbowax.

The composition of the invention may be incorporated into a medical device for administration. Such a device can include but is not limited to a fabric, patch, bandage, gauge, sock, tight, underwear, dressing, glove, mask, adhesive patches, non-adhesive patches, occlusive patches and microelectric patches or suitable adhesive system. In such an embodiment, the device is in direct contact with the keratinous layer such as the skin, thus releasing the peptides of the invention. It will be understood that the topical composition may be incorporated in any suitable form as detailed herein. For example, the topical composition or peptides of the invention can be incorporated into the device or be present on the surface of the device or can be in a cream, gel or wax formulation or any suitable formulation defined herein and incorporated into the device or on the surface of the device.

The device may be adapted for adhesion or attachment to the skin. In one embodiment, the device is adapted to release a constant quantity of the composition or the peptides of the invention. It will be understood that the amount of the composition contained in the sustained release system will depend, for example, on where the composition is to be administered, the kinetics and duration of the release of the composition of the invention, as well as the nature of the condition, disorder and/or disease to be treated and/or cared for. The device may be such that the composition is released by biodegradation of the device, or by friction between the device and the body, due to bodily moisture, the skin's pH or body temperature.

In an embodiment of the invention the composition may further comprise at least one cosmetically or pharmaceutically acceptable excipient. Excipient may be used interchangeably with functional ingredient or additive. It will be understood that although the topical compositions of the current invention can be administered alone, they will generally be administered in admixture with a cosmetic or pharmaceutical excipient. Cosmetically or pharmaceutically acceptable excipient are well known in the art and any known excipient, may be used provided that it is suitable for topical administration and is dermatologically acceptable without undue toxicity, incompatibility and/or allergic reaction.

Preferably any excipient included is present in trace amounts. The amount of excipient included will depend on numerous factors, including the type of excipient used, the nature of the excipient, the component(s) of the topical composition, the amount of active or peptide in the topical composition and/or the intended use of the topical composition. The nature and amount of any excipient should not unacceptably alter the benefits of the peptides of this invention.

In an embodiment of the invention the excipient may be a suitable diluent, carrier, binder, lubricant, suspending agent, coating agent, preservative, stabilisers, dyes, vehicle, solubilising agent, base, emollient, emulsifying agent, fragrance, humectant, and/or surfactants.

Examples of suitable diluents include, but are not limited to, any diluent disclosed in disclosed in US2014120131 or US2004132667. Examples include ethanol, glycerol and water. Examples of suitable carriers include, but are not limited to, lactose, starch, glucose, methyl cellulose, magnesium stearate, mannitol, sorbitol and any suitable carrier disclosed in US2014120131 or US2004132667.

Examples of suitable binders include, but are not limited to, starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol and any suitable binder disclosed in US2014120131 or US2004132667.

Examples of suitable lubricants include, but are not limited to, sodium oleate, sodium stearate, magnesium stearate, sodium benzoate, sodium acetate, and sodium chloride and any suitable lubricant disclosed in US2014120131 or US2004132667.

The carrier may be any suitable carried known in the art or disclosed in US2014120131 or US2004132667. In some embodiments, the carrier may include, but is not limited to, a liquid, such as water, oils or surfactants, including those of petroleum, animal, plant or synthetic origin, polymer, oil, such as peanut oil, mineral oil, castor oil, soybean oil, alcohol, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, or digitonin. It will be understood that the carrier will be dermatologically acceptable. Preferred carriers contain an emulsion such as oil-in-water, water-in-oil, water-in-oil-in-water and oil-in-water-in-silicone emulsions. Emulsions may further contain an emulsifier and/or an anti-foaming agent.

In an embodiment of the invention, the composition may further comprise one or more additional ingredients. The composition of the invention may be administered consecutively, simultaneously or sequentially with the one or more other additional agents. Such additional ingredients may be those of benefit to include in a topical composition, or of benefit depending on the intended use of the topical composition. The additional ingredient may be active or functional or both.

Examples of such additional ingredients include, but are not limited to, one or more of cleaning agents, conditioning agents, sunscreen, pigment, moisturiser, thickening agents, gelling agents, essential oil, astringents, pigments, anti-caking agent, anti-foaming agent, binders, additives, buffers, chelating agents, external analgesics, film formers or materials, bulking agents, polymers, opacifying agents, pH adjusters, propellants, reducing agents, sequestrants, skin bleaching and lightening agents, skin conditioning agents, aloe vera, healing agents, soothing agents, smoothing agents, pantothenic acid, treating agents, thickeners, vitamins. colourants, pharmaceuticals, antiseptic agents, antifoaming agents, buffering agents, astringents, polymers, pH adjuster, deodorant or any other dermatologically acceptable carrier or surfactant.

It is to be understood that additional ingredients listed may provide more than one benefit. The classification given herein is for clarity and convenience only and not intended to limit the additional ingredient to that particular application or category listed.

Any additional ingredients should be suitable for application to the skin without undue toxicity, incompatibility and/or allergic reaction.

In some embodiments, the additional ingredient has glucose transport activity or aids glucose transport activity. In some embodiments, the additional ingredient has anabolic activity. In some embodiments, the additional ingredient has anti-inflammatory activity or aids anti-inflammatory activity. In some embodiments, the additional ingredient has anti-aging activity or aids anti-aging activity. In some embodiments, the additional ingredient is for keratinous layer health and/or development, skin health and/or development, and/or muscle health, recovery and/or development. The active agent may be a pharmacological enhancer. Such active agents are known and available on the market. In such cases, the topical composition of the invention may be administered consecutively, simultaneously or sequentially with the one or more other active agents.

In some embodiments, the additional ingredient may be farnesol ([2E, 6E], -3, 7, 11,-trimethyl-2, 6, 10, dodecatrien-1-ol), phytantriol (3, 7, 11, 15, tetramethylhexadecane-1, 2, 3, -triol), desquamation actives, enzymes, enzyme inhibitors, enzyme activators, botanical extracts and marine extracts, anti-acne actives, anti-wrinkle or anti atrophy actives, anti-oxidant/radical scavengers, chelators, flavonoids, anti-inflammatory agents, anti-cellulite agents, topical anaesthetics, tanning actives, skin lightening agents, skin healing agents, bisabolol, antimicrobial or antifungal active, sunscreen actives, particulate material, conditioning agents, structuring agents, thickening agent. The desquamation active may be any suitable agent that enhances the skin appearance or texture of the skin and is as disclosed in US2014120131 or US2004132667.

Examples of anti-acne actives are as disclosed in US2014120131 or US2004132667 and include, resorcinol, salicylic acid, erythromycin, zine, sulfur, benzoyl peroxides.

Examples of thickening agents are as disclosed in US2014120131 or US2004132667 and include carboxylic acid polymers, crosslinked polyacrylate polymers, polyacrylamide polymers, polysaccharides.

Examples of conditioning agents are as disclosed in US2014120131 or US2004132667 and include humectants, moisturiser or skin conditioner.

Examples of structuring agents are as disclosed in US2014120131 or US2004132667 and include any agent that provide rheological characteristics to the composition and contributes to the stability of the composition.

Any suitable antimicrobial or antifungal active may be used and examples are as disclosed in US2014120131 or US2004132667. Such actives are capable of destroying microbes, preventing growth or action of microbes. Examples include but are not limited to β-lactam drugs, quinolone drugs, tetracycline, erythromycin, streptomycin sulfate, salicylic acid, benzoyl peroxide.

Examples of a particulate material include metallic oxide.

Examples of anti-cellulite agents include xanthine agents.

Examples of tanning actives includes 1, 3-dihydroxy-2-propanone and those disclosed in US2014120131 or US2004132667.

Examples of topical anaesthetics include benzocaine, lidocaine and bupivacaine and those disclosed in US2014120131 or US2004132667.

Examples of skin lightening agents include any agent known in the art such as kojic acid, ascorbic acid and those disclosed in US2014120131 or US2004132667.

Examples of sunscreen actives include any suitable organic or inorganic sunscreen active. Examples include metallic oxides, 2-ethylhexyl-p-methoxycinnamate and those disclosed in US2014120131 or US2004132667.

Examples of skin healing agents includes panthenoic acid as disclosed in US2014120131 or US2004132667.

Examples of anti-inflammatory agents include any agent that enhances the skin appearance, tone or colour and include but are not limited to corticosteroids, hydrocortisone, non-steroidal agents such as ibuprofen and aspirin and those disclosed in US2014120131 or US2004132667.

Examples of flavonoids includes flavanones, methoxy flavonones, unsubstituted chalcone and mixtures thereof and those disclosed in US2014120131 or US2004132667.

Examples of enzymes include lipases, proteases, catalase, super oxide-dismutase, amylase, peroxidase, glucuronidase, ceramidases, hyaluronidases. Examples of enzyme inhibitors include trypsine inhibitors, Bowmann Birk inhibitors, chymotrypsin inhibitors, botanical extracts, flavonoids, quercetin chalcone and those disclosed in US2014120131 or US2004132667 and mixtures thereof. Examples of enzyme activators include coenzyme A, Q10 (ubiquinone), glycyrrhizin, berberine, chrysin and those disclosed in US2014120131 or US2004132667 and mixtures thereof.

Examples of anti-wrinkle or anti atrophy actives include sulfur containing D and L amino acids, particular, N-acyl derivatives such as N-acetyl-L-cysteine, hydroxyl acids, phytic acid, lipoic acid, lysophosphatidic acid, skin peel agents, vitamin B₃, retinoids and those disclosed in US2014120131 or US2004132667 and mixtures thereof.

The anti-oxidant/radical scavenger agent may be any agent that is useful for providing protection against UV radiation or other environmental agents which may cause skin damage such as those disclosed in US2014120131 or US2004132667. Examples of anti-oxidant/radical scavengers include ascorbic acid, its salts and derivatives (vitamin C), tocopherol its salts and derivatives (vitamin E), butylated hydroxyl benzoic acids and their salts, peroxides, gallic acids and alkyl esters, sorbic acid, lipoic acid, amines, lycine pidolate, arginine pilolate, nordihydroguaiaretic acid, bioflavonoids, curcumin, lysine, methionine, proline, superoxide dismutase, silymarin, tea extracts and mixtures thereof.

Examples of chelators include EDTA, NTA, hydoxamic acids, phytic acid, lactoferrin and those disclosed in US2014120131 or US2004132667 and mixtures thereof. A chelator means an agent capable of removing a metal ion by forming a complex so that the metal ion cannot participate in or catalyse chemical reactions. A chelator is useful for protection against UV radiation or other environmental agents that can cause skin damage.

It will be appreciated that a plurality of additional ingredients may be added. The amount of the additional ingredient may be from about 0.001% to about 50% weight of the composition, preferably, about 0.01% to about 20%, preferably about 0.1% to about 10%, about 0.5% to about 10%, about 1% to about 5%, preferably 2% weight of the composition. The amount of additional ingredient included will depend on numerous factors, including the type of additional ingredient used, the nature of the additional ingredient, the component(s) of the topical composition, the amount of active or peptide in the topical composition and/or the intended use of the topical composition. The nature and amount of any additional ingredient should not unacceptably alter the benefits of the peptides of this invention.

The topical composition may be alcohol free.

In some embodiments of the invention, the composition further comprises one or more additional active agents, in addition to the peptide of the invention (also known as the active of the composition). In addition, or alternatively, the composition may be administered with one or more other additional active agents. Typical said additional active agent is present in trace amounts only. In some embodiments, there may be no additional active agent present in the composition. The amount of additional active agent included will depend on numerous factors, including the type of additional active agent used, the nature of the additional active agent, the component(s) of the topical composition, the amount of active or peptide in the topical composition and/or the intended use of the topical composition. The nature and amount of any additional active agent should not unacceptably alter the benefits of the peptides of this invention.

It is to be understood that an ingredient that is considered to be an "active" ingredient in one product may be a "functional" or "excipient" ingredient in another and vice versa. It will also be appreciated that some ingredients play a dual role as both an active ingredient and as a functional or excipient ingredient.

Examples of the additional active agents include glucose transport promoting drugs, skin supplement, agent for treatment and/or care of the skin, anti-inflammatory agent, an anti-aging agent, anabolic agents, a cellular growth promoting agent and pharmacological enhancers. Such agents are well known in the art and it will be appreciated that any suitable additional active agent may be used. Additional active agents for treatment and/or care of the skin may include collagen synthesis agents, retinoids, exfoliating agents, anti-cellulite agents, elastase inhibiting agents, melanin synthesis stimulating or inhibiting agents, self-tanning agents, antiaging agents, antimicrobial agents, antifungal agents, fungistatic agents, bactericidal agents, and healing agents. Active agents also include anti-inflammatory agents.

Any additional active agent should be suitable for application to the skin without undue toxicity, incompatibility and/or allergic reaction. It will be understood that the classification given herein is for clarity and convenience only and not intended to limit the additional ingredient, excipient, or active to that particular application or category listed.

In a particularly preferred embodiment, the methods and uses of the invention involve administration of a peptide or composition of the invention in combination with one or more other active agents, for example, existing growth promoting drugs or pharmacological enhancers available on the market. In such cases, the compounds of the invention may be administered consecutively, simultaneously or sequentially with the one or more other active agents.

In an embodiment, the effect of the current invention is accomplished by topical application or administration of the topical composition of the invention described herein to a person, animal or a patient in need of treatment or care. Topical delivery preferably means delivery to a keratinous layer such as the skin, hair and/or nails, but can also mean delivery to a body lumen lined with epithelial cells, for example the lungs or airways, the gastrointestinal tract, the buccal cavity. The effect may be confined to the surface of the skin or may be within the skin or a combination of both.

The topical composition of the invention is administered in a cosmetically or pharmaceutically effective amount. In other words, in an amount that is non-toxic but sufficient amount to provide the desired effect. It will be appreciated that a person skilled in the art would be capable of determining an appropriate dose of the topical compositions of the invention to administer without undue experimentation. Alternatively, a physician will determine the actual dose that is most suitable for a patient depending on the particular condition, disease or disorder to be treated or cared for and the age, body weight and/or health of the person. It will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. There can, of course, be individual instances where higher or lower dosage ranges are merited, and such are within the scope of this invention. For example, the composition may be administered at a dose of from 0.01 to 50 mg/kg body weight, such as from 0.1 to 30 mg/kg, more preferably from 0.1 to 20 mg/kg body weight, more preferably from 0.1 to 10 mg/kg body weight, preferably 0.1 to 5mg/kg body weight. In an exemplary embodiment, one or more doses of 10 to 300 mg/day or more preferably, 10 to 150 mg/day, will be administered to the patient. The amount and the frequency is as best suited to the purpose. The frequency of application or administration can vary greatly, depending on the needs of each subject, with a recommendation of an application or administration range from once a month to ten times a day, preferably from once a week to four times a day, more preferably from three times a week to three times a day, even more preferably once or twice a day.

In preferred embodiments, repeated use of the composition is provided.

The topical composition may be applied by, but not limited to, rubbing, or massaging into the keratinous tissue, skin or area of the body to be treated or cared for. In some embodiments, the composition is left on or not removed from the area of the body. In other embodiments, the composition is removed after a period of time, such as, but not limited to, from about 2 minutes to 60 minutes, from about 5 minutes to about 30 minutes, preferably from about 10 minutes to about 20 minutes. The composition may be removed immediately after application. In some embodiments of the current invention, the composition of the invention may be applied to an area to be treated by means to achieve a greater penetration of the composition and/or peptide of the invention, such as, but not limited to, iontophoresis, sonophoresis, electroporation, microelectric patches, mechanical pressure, osmotic pressure gradient, occlusive cure, microinjections or needle-free injections by means of pressure, such as injections by oxygen pressure, or any combination thereof.

The peptides of the invention are used in the topical cosmetic or pharmaceutical composition of this invention at cosmetically or pharmaceutically effective concentrations to achieve the desired effect; in a preferred form with regards to the total weight of the composition, between 0.00000001% (in weight) and 20% (in weight); preferably between 0.000001% (in weight) and 15% (in weight), more preferably between 0.0001% (in weight) and 10% (in weight) and even more preferably between 0.0001% (in weight) and 5% (in weight). Ideally, the peptides of the present invention are preferably used from about 0.00001% w/w to about 0.5% w/w, and more preferably from 0.00005 w/w to about 0.05 w/w, and most preferably from about 0.0001 w/w to about 0.01 w/w of the composition. Ideally, the peptides of the present invention are preferably used from about 0.0001% w/w to about 0.004% w/w of the composition.

The composition of the invention may be administered individually or in combination with other pharmacologically active agents. It will be understood that such combination therapy encompasses different therapeutic regimens, including, without limitation, administration of multiple agents together in a single dosage form or in distinct, individual dosage forms. If the agents are present in different dosage forms, administration may be simultaneous or near-simultaneous or may follow any predetermined regimen that encompasses administration of the different agents. The suitable active agents may be as described herein.

In some embodiments of the current invention, the composition may be delivered via any one of liposomes, mixed liposomes, oleosomes, niosomes, ethosomes, millicapsules, capsules, macrocapsules, nanocapsules, nanostructured lipid carriers, sponges, cyclodextrins, vesicles, micelles, mixed micelles of surfactants, surfactant-phospholipid mixed micelles, millispheres, spheres, lipospheres, particles, nanospheres, nanoparticles,milliparticles, solid nanopartciles as well as microemulsions including water-in-oil microemulsions with an internal structure of reverse micelle and nanoemulsions microspheres, microparticles.

A variety of methods are available for preparing liposomes. See, e.g., Szoka et al., Ann. Rev. Biophys. Bioeng. 9:467 (1980), U.S. Pat. Nos. 4,186,183, 4,217,344, 4,235,871, 4,261,975, 4,485,054, 4,501,728, 4,774,085, 4,837,028, 4,235,871, 4,261,975, 4,485,054, 4,501,728, 4,774,085, 4,837,028, 4,946,787, PCT Publication No. WO 91/17424, Deamer & Bangham, Biochim. Biophys. Acta 443:629-634 (1976); Fraley, et al., PNAS 76:3348-3352 (1979); Hope et al., Biochim. Biophys. Acta 812:55-65 (1985); Mayer et al., Biochim. Biophys. Acta 858:161-168 (1986); Williams et al., PNAS 85:242-246 (1988); Liposomes (Ostro (ed.), 1983, Chapter 1); Hope et al., Chem. Phys. Lip. 40:89 (1986); Gregoriadis, Liposome Technology (1984) and Lasic, Liposomes: from Physics to Applications (1993)). Suitable methods include, for example, sonication, extrusion, high pressure/homogenization, microfluidization, detergent dialysis, calcium-induced fusion of small liposome vehicles and ether fusion methods, all of which are well known in the art.

These delivery systems may be adapted to achieve a greater penetration of the compound and/or peptides of the invention. This may improve pharmacokinetic and pharmacodynamics properties. The delivery system may be a sustained release system wherein the compound or peptide of the invention is gradually released during a period of time and preferably with a constant release rate over a period of time. The delivery systems are prepared by methods known in the art. The amount of peptide contained in the sustained release system will depend on where the composition is to be delivered and the duration of the release as well as the type of the condition, disease and/or disorder to be treated or cared for.

The composition of the invention may be for human or animal usage in human and veterinary medicine.

The composition of the invention may be used for pharmaceutical, personal care and/or cosmetic uses.

The composition can be used to treat or care for any disease, disorder or condition of the skin, including but not limited to, psoriasis, dermatitis, allergic dermatitis, eczema, spongiosis, edema, skin cancer, ulcers, acne, scars, cellulitis, elastosis, keratosis, rosacea, varicose veins, inflammatory disorders.

The composition may be used to for treating or caring for visible signs of aging including but not limited to wrinkles, stretch marks and dark circles, dryness, fine lines, age spots, red blotches, sagging skin, and conditions caused by sun exposure including sunburn, stress, pollution and/diet. The topical composition may also be used for delaying, slowing or inhibiting the skins or the onset of aging. The composition may be administered by a medical device, such as a plaster or a patch as described herein.

The composition may be used to treat or care for a wound in a mammal. In another embodiment, the topical composition is for use in the treatment or prevention of a disease or condition characterised by damaged epithelial cells or tissue, and/or damaged dermal or epithelial cells or tissue. The disease may be but is not limited to cancer and trauma.

The composition may be used to treat or care for any muscle condition, to improve, muscle status in a mammal, to promote recovery of muscle, typically following exercise, to maintain or restore muscle health (for example lean tissue mass) in a mammal, to enhance physical performance, in treatment or prevention of a disease or condition characterised by lethargy or low energy levels.

The composition may be used to increase or stimulate muscle growth.

The composition may be used to promote growth of a tissue, promote growth of epithelial tissue, promote growth of skin, promote growth of an organ, promote growth of an organism. The skin can have a normal pathology and/or an abnormal pathology.

The composition may also be used to treat or care for any inflammatory disorder. In one embodiment, the inflammatory disorder is an inflammatory disorder of the joints. In one embodiment, the inflammatory disorder is dermatitis. In one embodiment, the inflammatory disorder is acne vulgaris. In one embodiment, the inflammatory disorder is psoriasis. In one embodiment, the inflammatory disorder is rheumatoid arthritis.

In an embodiment of the invention the composition is for use in a method for the treatment or prevention of local pain.

The composition has a use as a personal care product, a supplement, a cosmetic, a pharmaceutical product.

A method of treating, preventing or caring for any one of the diseases, disorders or conditions described herein is also provided, said method comprising a step of administering the topical composition of the invention. The composition may be administered to the skin, hair, the nails. The composition may be administered in any dose or frequency as disclosed herein by any method of topical application.

In one embodiment, the composition is a cosmetic composition. In one embodiment, the composition is a pharmaceutical composition. It will be appreciated that the composition may have a dual function and be both a cosmetic and pharmaceutical composition.

It will be appreciated that the composition may be a therapeutic composition or may be a non-therapeutic composition. The composition may have a dual role.

In an embodiment of the invention the peptide or peptide variant may be a modified peptide. The term "modified peptide" is used interchangeably with the term derivative of the peptide. The modified peptide includes but is not limited to a peptide which has been substituted with one or more groups as defined herein.

In one embodiment, the modification may be any modification that provides the peptides and or the composition of the invention with an increased ability to penetrate a cell. In one embodiment, the modification may be any modification that increases the half-life of the composition or peptides of the invention. In one embodiment, the modification may be any modification that increases activity of the composition or peptides of the invention. In one embodiment, the modification may be any modification that increases selectivity of the composition or peptides of the invention.

In one embodiment, the group is a protecting group. The protecting group may be an N-terminal protecting group, a C-terminal protecting group or a side-chain protecting group. The peptide may have one or more of these protecting groups.

The person skilled in the art is aware of suitable techniques to react amino acids with these protecting groups. These groups can be added by preparation methods known in the art, for example the methods as outlined in paragraphs [0104] to [0107] of US2014120141. The groups may remain on the peptide or may be removed. The protecting group may be added during synthesis.

In an embodiment of the invention the peptides may be substituted with a group selected from one or more straight chain or branched chain, long or short chain, saturated, or unsaturated, substituted with a hydroxyl, amino, amino acyl, sulfate or sulphide group or unsubstituted having from 1 to 29 carbon atoms. N-acyl derivatives include acyl groups derived from acetic acid, capric acid, lauric acid, myristic acid, octanoic acid, palmitic acid, stearic acid, behenic acid, linoleic acid, linolenic acid, lipoic acid, oleic acid, isosteric acid, elaidoic acid, 2-ethylhexaneic acid, coconut oil fatty acid, tallow fatty acid, hardened tallow fatty acid, palm kernel fatty acid, lanolin fatty acid or similar acids. These may be substituted or unsubstituted. When substituted, they are preferably substituted with hydroxyl, or sulphur containing groups such as but not limited to SO₃H, SH, or S-S.

In an embodiment of the current invention, the peptide is R₁-X- R₂. R₁ and/or R₂ groups respectively bound to the amino-terminal (N-terminal) and carboxyl-terminal (C-terminal) of the peptide sequence.

In one embodiment, the peptide is R₁-X. Alternatively, the peptide is X- R₂. Preferably, R₁ is H, C₁₋₄ alkyl, acetyl, benzoyl or trifluoroacetyl; X is the peptide of the invention; R₂ is OH or NH₂.

In an embodiment, R₁ is selected from the group formed by H, a non-cyclic substituted or unsubstituted aliphatic group, substituted or unsubstituted alicyclyl, substituted or unsubstituted heterocyclyl, substituted or unsubstituted heteroarylalkyl, substituted or unsubstituted aryl, substituted or unsubstituted aralkyl, Tert-butyloxycarbonyl, 9-fluorenylmethyloxycarbonyl (Fmoc) and R₅-CO-, wherein R₅ is selected from the group formed by H, a non-cyclic substituted or unsubstituted aliphatic group, substituted or unsubstituted alicyclyl, substituted or unsubstituted aryl, substituted or unsubstituted aralkyl, substituted or unsubstituted heterocyclyl and substituted or unsubstituted heteroarylalkyl; R₂ is selected from the group formed by -NR₃R₄, -OR₃ and -SR₃, wherein R₃ and R₄ are independently selected from the group formed by H, a non-cyclic substituted or unsubstituted aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl; and with the condition that R₁ and R₂ are not α-amino acids.

In accordance with another preferred embodiment, R₂ is -NR₃R₄, -OR₃ or -SR₃ wherein R₃ and R₄ are independently selected from the group formed by H, substituted or unsubstituted C₁-C₂₄ alkyl, substituted or unsubstituted C₂-C₂₄ alkenyl, Tert-butyloxycarbonyl, 9-fluorenylmethyloxycarbonyl (Fmoc), substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3-10 members, and substituted or unsubstituted heteroarylalkyl of 2 to 24 carbon atoms and 1 to 3 atoms other than carbon wherein the alkyl chain is of 1 to 6 carbon atoms. Optionally, R₃ and R₄ can be bound by a saturated or unsaturated carbon-carbon bond, forming a cycle with the nitrogen atom. More preferably R₂ is -NR₃R₄ or -OR₃, wherein R₃ and R₄ are independently selected from the group formed by H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₁₀ cycloalkyl, substituted or unsubstituted C₆-C₁₅ aryl and substituted or unsubstituted heterocyclyl of 3-10 members, substituted or unsubstituted heteroarylalkyl with a ring of 3 to 10 members and an alkyl chain of 1 to 6 carbon atoms. More preferably R₃ and R₄ are selected from the group formed by H, methyl, ethyl, hexyl, dodecyl, or hexadecyl. Even more preferably R₃ is H and R₄ is selected from the group formed by H, methyl, ethyl, hexyl, dodecyl, or hexadecyl. In accordance with an even more preferred embodiment, R₂ is selected from -OH and -NH₂.

In accordance with another embodiment of this invention R₁ is selected from the group formed by H, acetyl, lauroyl, myristoyl or palmitoyl, and R₂ is -NR₃R₄ or -OR₃ wherein R₃ and R₄ are independently selected from H, methyl, ethyl, hexyl, dodecyl and hexadecyl, preferably R₂ is -OH or -NH₂. More preferably, R₁ is acetyl or palmitoyl and R₂ is -NH₂.

In a preferred embodiment, the acyl group is bound to the N-terminal end of at least one amino acid of the peptide.

In an embodiment of the invention, the peptide is modified to comprise a side chain protecting group. The side chain protecting group may be one or more of the group comprising benzyl or benzyl based groups, t-butyl-based groups, benzyloxy-carbonyl (Z) group, and allyloxycarbonyl (alloc) protecting group. The side chain protecting group may be derived from an achiral amino acid such as achiral glycine. The use of an achiral amino acid helps to stabilise the resultant peptide and also facilitate the facile synthesis route of the present invention. Preferably, the peptide further comprises a modified C-terminus, preferably an amidated C-terminus. The achiral residue may be alpha-aminoisobutyric acid (methylalaine). It will be appreciated that the specific side chain protecting groups used will depend on the sequence of the peptide and the type of N-terminal protecting group used.

In one embodiment of the invention the peptide is conjugated, linked or fused to one or more polyethylene glycol polymers or other compounds, such as molecular weight increasing compounds. The molecular weight increasing compound is any compound that will increase the molecular weight, typically by 10% to 90%, or 20% to 50% of the resulting conjugate and may have a molecular weight of between 200 and 20,000, preferably between 500 and 10,000. The molecular weight increasing compound may be PEG, any water-soluble (amphiphilic or hydrophilic) polymer moiety, homo or co-polymers of PEG, a monomethyl-subsitututed polymer of PEG (mPEG) and polyoxyethylene glycerol (POG), polyamino acids such as poly-lysine, poly-glutamic acid, poly-aspartic acid, particular those of L conformation, pharmacologically inactive proteins such as albumin, gelatin, a fatty acid, olysaccharide, a lipid amino acid and dextran. The polymer moiety may be straight chained or branched and it may have a molecular weight of 500 to 40000Da, 5000 to 10000 Da, 10000 to 5000 Da. The compound may be any suitable cell penetrating compound, such as tat peptide, penetratin, pep-1. The compound may be an antibody molecule. The compound may be a lipophilic moiety or a polymeric moiety.

The lipophilic substituent and polymeric substituents are known in the art. The lipophilic substituent includes an acyl group, a sulphonyl group, an N atom, an O atom or an S atom which forms part of the ester, sulphonyl ester, thioester, amide or sulphonamide. The lipophilic moiety may include a hydrocarbon chain having 4 to 30 C atoms, preferably between 8 and 12 C atoms. It may be linear or branched, saturated or unsaturated. The hydrocarbon chain may be further substituted. It may be cycloalkane or heterocycloalkane. The peptide may be modified at the N-terminal, C-terminal or both. The polymer or compound is preferably linked to an amino, carboxyl or thio group and may be linked by N-termini or C-termini of side chains of any amino acid residue. The polymer or compound may be conjugated to the side chain of any suitable residue.

The polymer or compound may be conjugated via a spacer. The spacer may be a natural or unnatural amino acid, succinic acid, lysyl, glutamyl, asparagyl, glycyl, beta-alanyl, gamma-amino butanoyl.

The polymer or compound may be conjugated via an ester, a sulphonyl ester, a thioester, an amide, a carbamate, a urea, a sulphonamide.

A person skilled in the art is aware of suitable means to prepare the described conjugate.

Peptides can be chemically modified by covalent conjugation to a polymer to increase their circulating half-life, for example. Exemplary polymers and methods to attach such polymers to peptides are illustrated in, *e.g.,* U.S. Pat. Nos. 4,766,106; 4,179,337; 4,495,285; and 4,609,546. Additional illustrative polymers include polyoxyethylated polyols and polyethylene glycol (PEG) moieties.

The peptides of the invention may be subjected to one or more modifications for manipulating storage stability, pharmacokinetics, and/or any aspect of the bioactivity of the peptide, such as, *e.g.,* potency, selectivity, and drug interaction. Chemical modification to which the peptides may be subjected includes, without limitation, the conjugation to a peptide of one or more of polyethylene glycol (PEG), monomethoxy-polyethylene glycol, dextran, poly-(N-vinyl pyrrolidone) polyethylene glycol, propylene glycol homopolymers, a polypropylene oxide/ethylene oxide co-polymer, polypropylene glycol, polyoxyethylated polyols (e.g., glycerol) and polyvinyl alcohol, colominic acids or other carbohydrate based polymers, polymers of amino acids, and biotin derivatives. PEG conjugation of proteins at Cys residues is disclosed, *e.g*., in Goodson, R. J. & Katre, N. V. (1990) Bio/Technology 8, 343 and Kogan, T. P. (1992) Synthetic Comm. 22, 2417.

Modified peptides also can include sequences in which one or more residues are modified (*i.e.,* by phosphorylation, sulfation, acylation, PEGylation, etc.), and mutants comprising one or more modified residues with respect to a parent sequence. Amino acid sequences may also be modified with a label capable of providing a detectable signal, either directly or indirectly, including, but not limited to, radioisotope, fluorescent, and enzyme labels. Fluorescent labels include, for example, Cy3, Cy5, Alexa, BODIPY, fluorescein (*e.g*., FluorX, DTAF, and FITC), rhodamine (*e.g*., TRITC), auramine, Texas Red, AMCA blue, and Lucifer Yellow. Preferred isotope labels include ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, and ²⁸⁶Re. Preferred enzyme labels include peroxidase, β-glucuronidase, β-D-glucosidase, β-D-galactosidase, urease, glucose oxidase plus peroxidase, and alkaline phosphatase (see, *e.g.,* U.S. Pat. Nos. 3,654,090; 3,850,752 and 4,016,043). Enzymes can be conjugated by reaction with bridging molecules such as carbodiimides, diisocyanates, glutaraldehyde, and the like. Enzyme labels can be detected visually, or measured by calorimetric, spectrophotometric, fluorospectrophotometric, amperometric, or gasometric techniques. Other labeling systems, such as avidin/biotin, Tyramide Signal Amplification (TSA™), are known in the art, and are commercially available (see, *e.g.,* ABC kit, Vector Laboratories, Inc., Burlingame, Calif.; NEN® Life Science Products, Inc., Boston, Mass.).

In an embodiment, the peptide, variant and/or composition is modified to increase drug performance ability. In an embodiment, the peptide, variant and/or composition is modified to increase stability, permeability, maintain potency, avoid toxicity and/or to increase half-life. The modification may be as described above. For example, the modification may be to protect the N and C-terminus, it may be a modified amino acid, cyclisation, replacement of an amino acid, and/or conjugation to macromolecules or large polymers or long life plasma proteins. Strategies to extend a half-life may be as described by Strohl, et al (BioDrugs, 2015), Schlapschy, et al (Protein Eng Des Sel. 2013), Podust, VN, et al (Protein Eng Des Sel. 2013), Zhang, L et al (Curr Med Chem. 2012), Gaberc-Porekar, V, et al (Curr Opin Drug Discov Devel. 2008). Examples include using PEGylation, lipidation (covalent binding of fatty acids to peptide side chains), fusion to Fc domains and human serum albumin, fusion with a hydrophilic amino acid polymer, *e.g*. XTEN or PAS, and/or fusion with half-life extension proteins.

Compositions may be formulated in unit dosage form, *i*.*e*., in the form of discrete portions containing a unit dose, or a multiple or sub-unit of a unit dose.

In a particularly preferred embodiment, the methods and uses of the invention involve administration of a peptide or composition of the invention in combination with one or more other active agents available on the market. In such cases, the compounds of the invention may be administered consecutively, simultaneously or sequentially with the one or more other active agents.

### EXAMPLES

The peptide referred to in Example 1 to is a peptide having a sequence of SEQ ID NO.1.

### EXAMPLE 1

### Cell Proliferation Assay

### Study Description

BrDu is incorporated into newly synthesised DNA strands of actively proliferating cells. Following partial denaturation of double stranded DNA, BrDu is detected immunochemically allowing the assessment of the population of cells which are synthesizing DNA.

### Methodology

Human Dermal Fibroblasts (HDF - Sigma 10605a) were seeded in a 96 well plate at 10,000 cells per well in DMEM containing 10% fetal calf serum (FCS), 1% Pen/strep, 1% L-glutamine and allowed to adhere for 24h.

Following the initial 24h incubation the cells were incubated with 5 µg/ml, 0.5 µg/ml or 0.05µg/ml synthetic peptide for 24h respectively.

After 18 h incubation with synthetic peptides 20 µl BrDu reagent was added to each well. At 24h incubation the cells were fixed and the amount of 2-DG6P was measured using the BrdU Cell Proliferation Assay, all steps were carried out according to the manufacturer's instructions.

This experiment was then repeated using HACAT cells.

### Results

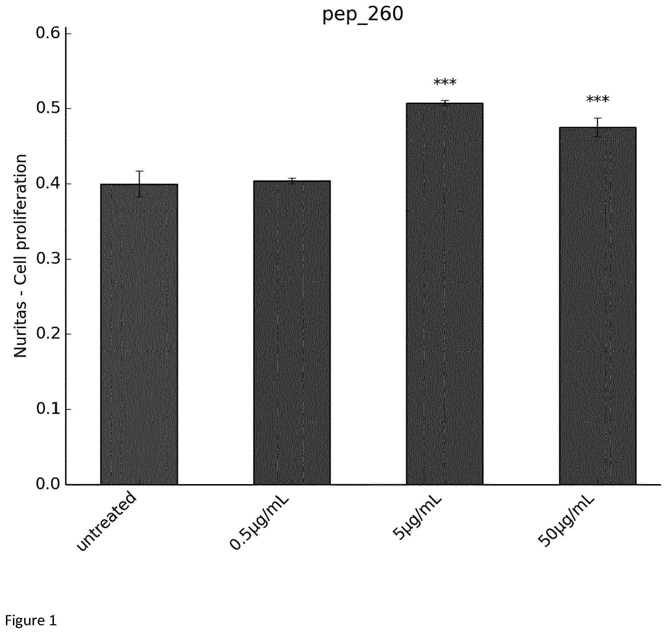

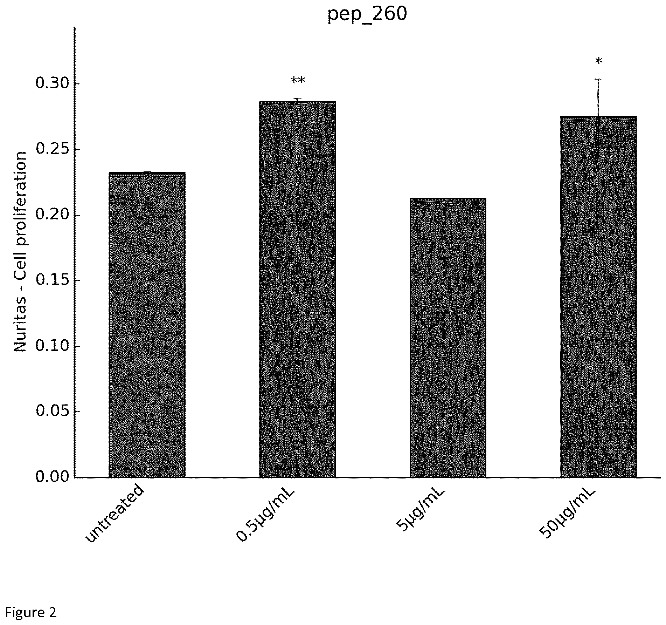

The results were calculated as a percentage of the untreated control. An increase in optical density reading indicates greater incorporation of BrDu and increase cell proliferation as illustrated by Figures 1 (HDF cells) and 2 (HACAT cells). As illustrated by Figures 1 and 2, samples stimulated with the peptide of the invention showed an increase in cell proliferation compared with untreated control.

### Conclusion

These results demonstrate the efficacy of the peptide to facilitate cell proliferation.

### EXAMPLE 2

### Collagen Production Assay

### Study Description

Hydroxyproline in tissue preparations is a direct measure of the amount of collagen present. FIRELISA Human Hydroxyproline ELISA kit assay is designed to measure hydroxyproline in tissue or peptide compositions.

### Methodology

Human Dermal Fibroblasts (HDF Sigma 10605a) were seeded in 24 well plates at 50,000 cells per well in DMEM containing 10% fetal calf serum (FCS), 1% Pen/strep, 1% L-glutamine and allowed to adhere for 24h.

Following the initial 24h incubation the cells were incubated with 5 µg/ml, 1 µg/ml or 0.1µg/ml synthetic peptide for 96h respectively.

After treatment, the cell supernatant was removed and centrifuged. 50 µl of each supernatant was assayed using the FIRELISA Human Hydroxyproline ELISA kit. All steps were carried out according to the manufacturer's instructions.

### Results

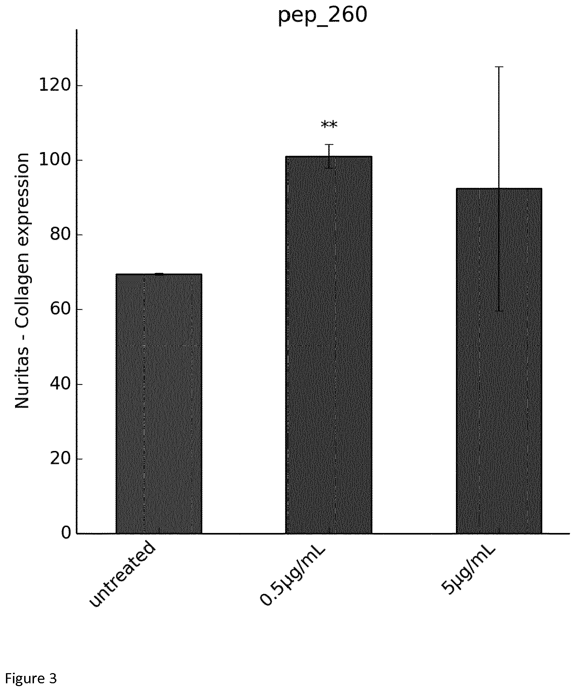

Results were calculated as a percentage of the untreated control. An increase in optical density reading indicates an increase collagen content. An increase in collagen production compared with untreated control was seen as illustrated by Figure 3.

### Conclusion

These results demonstrate the efficacy of the peptide to facilitate collagen production.

### EXAMPLE 3

### Elastin Production Assay

### Study Description

Elastin is a highly elastic protein in connective tissue and allows many tissues in the body to resume their shape after stretching or contracting. FIRELISA Human Elastin ELISA kit assay is designed to measure Elastin in tissue or protein/peptide compositions.

### Methodology

Human Dermal Fibroblasts (HDF) were seeded in 24 well plates at 50,000 cells per well in DMEM containing 10% fetal calf serum (FCS), 1% Pen/strep, 1% L-glutamine and allowed to adhere for 24h.

Following the initial 24h incubation the cells were incubated with 5 µg/ml, 1 µg/ml or 0.1µg/ml synthetic peptide for 96h respectively.

After treatment, the cells were lysed using cell lysis buffer and sonicated for 10 seconds. The lysed cells were centrifuged and the supernatant was removed from each sample. Total protein concentration was determined using a BCA assay. Each sample was diluted in assay buffer to contain 10 µg total protein. 50 µl of each sample was then assayed using the FIRELISA Human Elastin ELISA kit. All steps were carried out according to the manufacturer's instructions.

### Results

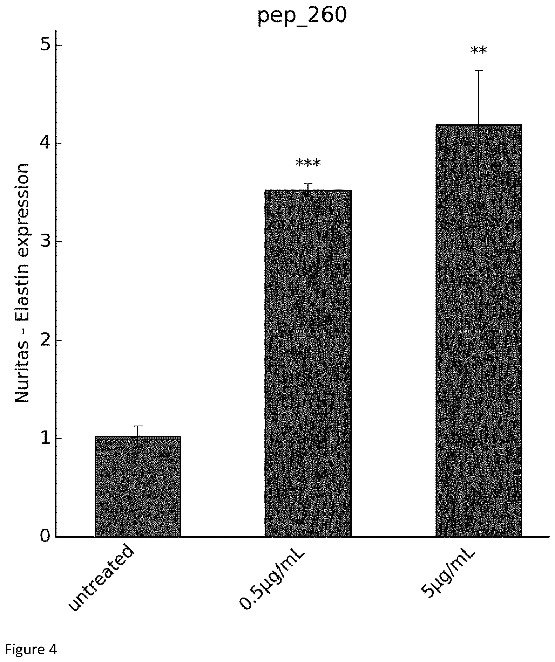

The results were calculated as a percentage of the untreated control. An increase in optical density reading indicates an increase elastin content. As illustrated by Figure 4, samples stimulated with the peptide of the invention showed an increase in elastin production compared with untreated control.

### Conclusion

These results demonstrate the efficacy of the peptide to facilitate elastin production.

### EXAMPLE 4

### Glucose Uptake

### Study Description

Glucose uptake in skeletal muscle cells was measured using 2-deoxyglucose (2-DG). 2-DG is taken up by glucose transporters and metabolized to 2-DG-6-phosphate (2-DG6P). The amount of accumulated non-metabolized 2-DG6P is proportional to glucose uptake by cells.

### Methodology

Human skeletal myoblasts (Sigma 150-05a) were seeded in a 96 well plate at 10,000 cells per well in Skeletal Muscle Differentiation medium and allowed to differentiated for 72h prior to experimentation.

The differentiated cells were serum starved for 24h prior to stimulation with insulin or synthetic peptides. After starvation, the serum free media was removed, cells rinsed with Phosphate Buffered Saline (PBS) and media replaced with 100 µl of Krebs-Ringer-Phosphate-HEPES (KRPH) and incubated for 1h.

The cells were then stimulated with 100nM insulin for 30 minutes or 5 µg/ml, 0.5 µg/ml or 0.05µg/ml synthetic peptide for 3h respectively.

Following stimulation, the cells were incubated with 10 µl/well of 2-DG solution for 40 min and glucose uptake was measured using the 'PrismColor Glucose Uptake Assay Kit' (Molecutools), all steps were carried out according to the manufacturer's instructions. The experiment was repeated using HACAT cells.

### Results

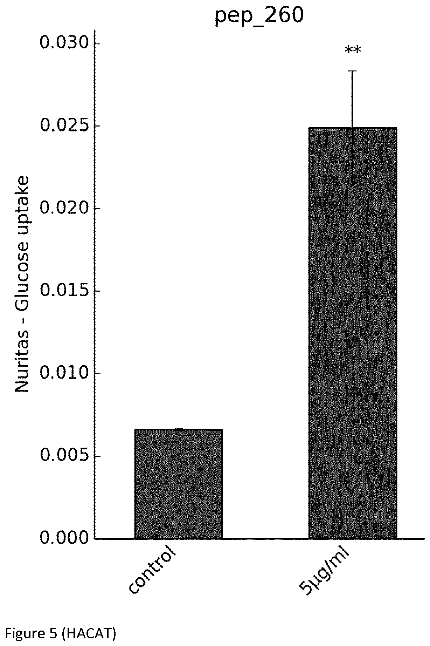

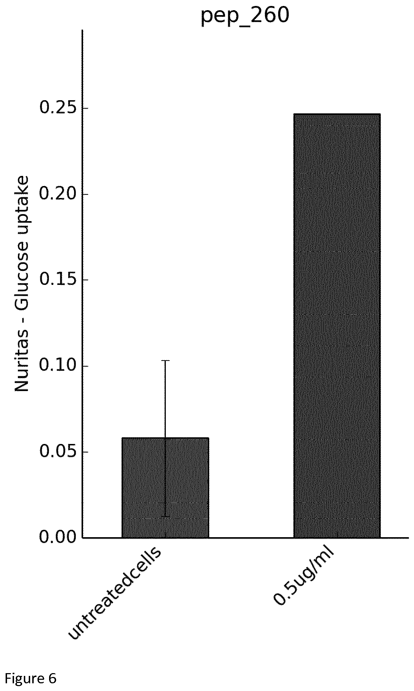

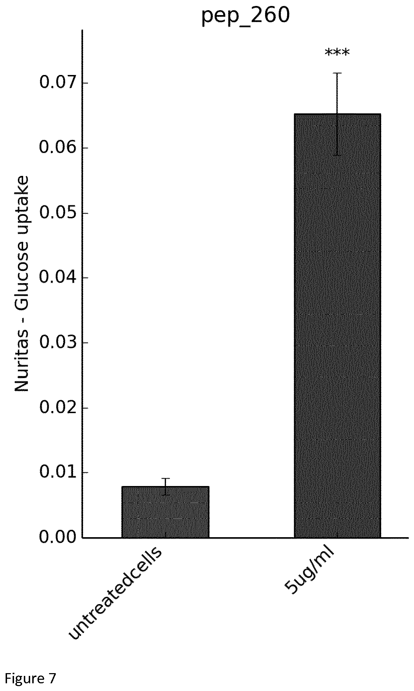

The results were calculated as a percentage of the untreated control. An increase in optical density reading indicates greater incorporation of 2-DG6P and increase in glucose uptake. All experiments were carried out in duplicate on three plates (6 wells/condition). Significance was determined using the Students t-test (*p<0.05 compared to control, **p<0.01 compared to control, *** p<0.001 compared to control). Figure 6 illustrates the results for 0.5 µg/ml synthetic peptide and Figure 7 illustrates results for 5 µg/ml synthetic peptide. Figure 5 displays the results for HACAT cells. As illustrated by these Figures, samples stimulated with the peptide of the invention showed an increase in glucose uptake compared with untreated control.

### Conclusion

These results demonstrate the efficacy of the peptide to facilitate glucose uptake in skeletal muscle.

### EXAMPLE 5

### GLUT-4 Translocation

### Methodology

HSKMC cells sere cultured in 6-well plates and then treated with peptides/hydrolysates for 2 hrs, at concentrations of 0.5ug/ml and 5ug/ml. Upon completion of treatment, cell membrane proteins were collected. Briefly, cells were harvested from the wells with a cell scraper and centrifuged at 300 x g for 5 min. The cell pellet was washed with 300ul of cell wash solution and then re centrifuged at 300 x g for 5 min. The supernatant was discarded and the cells resuspended in 1.5ml of cell wash solution and transferred to a 2ml centrifuge tube. The cells were then centrifuged at 300 x g for 5 min and the supernatant discarded. 150ul of permeabilization buffer was added to the cell pellet and vortexed briefly to obtain a homogenous cell suspension. The suspension was then incubated at 4°C for 10 min with constant mixing. Permeabilized cells were then centrifuged at 16000 x g for 15 min and the supernatant containing the cytosolic proteins was transferred to a new tube. 100ul of solubilisation buffer was added to the pellet and resuspended by gentle pipetting. The samples were then incubated at 4°C for 10 min with constant mixing. The samples were then centrifuged at 16000 x g for 15 min at 4°C for 10 min. The supernatant containing solubilized membrane and membrane associated proteins fractions was transferred to a new tube and stored at -80°C until ready for assessment.

GLUT-4 was assessed in the cell membrane fractions using a sandwich ELISA from LSBio. 10ug of protein from each sample was loaded into an individual well on a 96-well plate to a final volume of 100ul. GLUT-4 standards (0.3ng/ml- 20ng/ml) were also added to the plates at an identical volume. Plates were then sealed and incubated at 37°C for 1 hour. The liquid was aspirated from each well and then 100ul of detection reagent A was added to each well, at which point the plates were sealed, gently agitated to ensure mixing and then incubated for 1 hour at 37°C. The liquid was aspirated from each well and wells were washed 3 times using 350ul of wash buffer. 100ul of detection reagent B was added to each well, at which point the plates were sealed and incubated at 37°C for 30 min. The wells were washed 5 times, as outlined previously, and 90ul of TMB substrate was added to each well. The plates were sealed, protected from light, and incubated at 37°C for 10 minutes to ensure optimal colour development. 50ul of stop solution was added to each well and the optical density of the sample was determined using a microplate reader set to 450nm. GLUT-4 concentration in the samples was calculated off the standard curve after generating a four-parameter logistic (4-PL) curve fit. Sample concentration read of the curve was then adjusted by multiplying the results by the dilution factor.

### Results

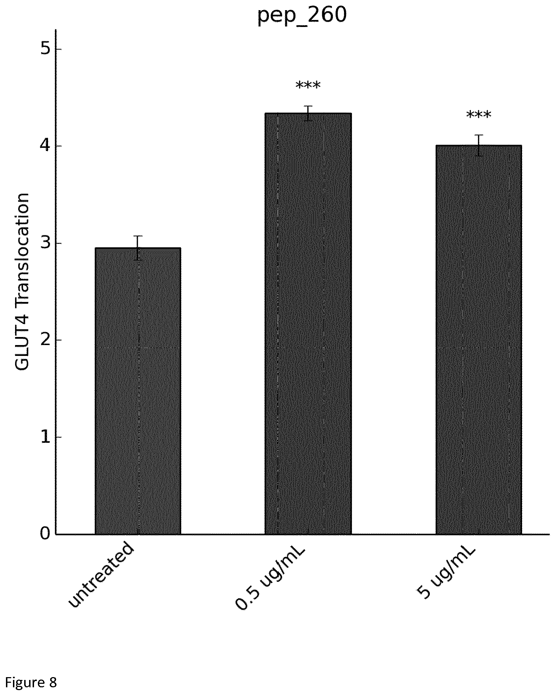

As illustrated by Figure 8, there was an increase in GLUT4 translocation in samples stimulated with both .05ug/ml and 5ug/ml peptide. The peptide of the invention displayed

### Conclusion

The peptide displayed a trend for stimulatory effect on skeletal muscle GLUT4 translocation and its ability to facilitate glucose transport in skeletal muscle.

### EXAMPLE 6

### Muscle Protein Synthesis (Phospho-mTOR assay)

### Study Description

The mammalian target of rapamycin (mTOR) is a Ser/Thr protein kinase that functions as an ATP and amino acid sensor to balance nutrient availability and cell growth. The activity of mTOR is regulated by insulin, amino acids, exercise, oxidative stress and growth factors which throughout its phosphorylation promote protein synthesis pathways. However, dysregulated mTOR cause accelerated ageing among many other pathologies. In muscle, when mTOR is activated (phosphorylated) leads skeletal muscle hypertrophy. This fact has been widely reported, in both rodents and humans, in the literature. Amino acids such as leucine enhances the phosphorylation of mTOR, and its activation up-regulates protein translation through the phosphorylation of the eukaryotic initiation factor 4E binding protein 1 (4E-BP1) and the ribosomal protein S6 kinase (S6K), leading to cell growth and proliferation.

Phospho-mTOR sandwich ELISA detects endogenous levels of phosphorylated mTOR at Ser2448.

### Methodology

**Day 1:** Seed HSkMC cells in 500uL/well of HSkMC Growth Medium and let them adhere to the wells for 16-24h.
**Day 2:** Remove growth medium and add 500uL/well of HSkMC Differentiation Medium to let them differentiate for 5-7 days.
**Day 7:** Treat cells for 2 hours. Collect cell lysates in lysis buffer.
**Day 8:** Perform BCA (protein determination) and run the assay. In a first incubation step, a mTOR antibody coated onto the ELISA microwells is used to capture mTOR (phospho and non-phospho) from the cell lysates. Then, following extensive washing, a specific phospho-mTOR detection antibody is added to detect only the phospho-mTOR protein captured before. Finally, a HRP-linked antibody is used to recognise the detection antibody and through the addition of TMB (HRP substrate) develop color proportionally to the quantity of mTOR phosphorylated at Ser2448.

### Results

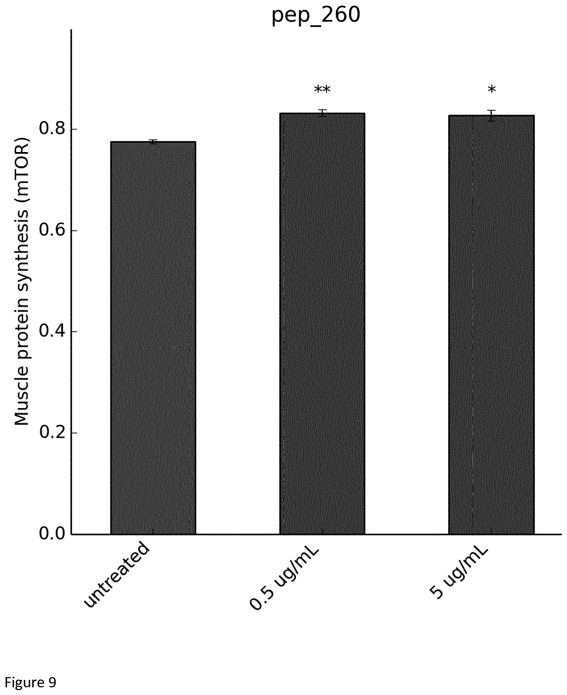

The results are illustrated by Figure 9. Samples stimulated with 0.5 µg/ml and 5µg/ml displayed in increase in mTOR.

### Conclusion

These results demonstrate the efficacy of the peptide to facilitate muscle protein synthesis and muscle growth.

### EXAMPLE 7 to 15: Formulations

In an embodiment, the composition is formulated as a topical composition. In one embodiment, it may be an emulsion or cream or a rub, such as a muscle rub.

In an embodiment, the cream comprises an excipient or diluent, a suspending agent, a preservative and an amount of at least one peptide of the invention.

In an embodiment, the cream comprises an alcohol, a carbomer, a sorbate, water and at least one peptide of the invention.

Preferably the alcohol is butylene glycol (1,3-butanediol). The alcohol may be present in an amount of between 1 and 10 % of the composition, preferably between 2 and 6%, preferably 4%. The sorbate may be polysorbate-20. The sorbate may be present in an amount of between 0.01 to 1% of the composition, preferably between 0.05 and 0.5%, preferably 0.10%. Water is present in an amount of between 10% and 90%, preferably 30% to 75%. The carbomer may be present in an amount of from 0.05% to 1%, preferably, 0.1% to 0.5%, preferably, 0.15%. The carbomer may be Ultrez 10.

The peptide of the invention may be present in an amount of from 0.5% to 10%, preferably, 1% to 5%, preferably 3%.

The composition may further comprise one or more of sugar alcohol such as glycerine, parabens, silicon, such as cyclohexasiloxane, a fatty alcohol or phosphoric acid or a mixture of fatty alcohol and phosphoric acid, such as cetearyl alcohol, dicetyl phosphate and Cereth 10 phosphate or combinations thereof, a polyoxyethylene stearyl ethers, such as Steareth 2 and 10, and a fragrance.

An exemplary rub or emulsion is follows in Example 7.

| **Ingredient** | **Exemplary (w/w)%** |
|---|---|
| **Phase A** | |
| Water | 70.95 |
| Carbomer | 0.15 |
| **Phase B** | |
| Glycerin | 3.5 |
| **Phase C** | |
| Steareth 2 | 0.40 |
| Cetearyl alcohol dicetyl phosphate and Ceteth 10 phosphate | 4 |
| Cyclohexsiloxane | 2 |
| Dioctyl succinate | 7 |
| Steareth 10 | 1.2 |
| Mixed parabens | 0.30 |
| **Phase D** | |
| Sorbate | 0.10 |
| **Phase E** | |
| Water | 2.50 |
| Sodium hydroxide | 0.30 |
| **Phase F** | |
| Fragrance | 0.10 |
| **Phase G** | |
| Peptide(s) of the invention | 3 |

The resulting emulsion is suited as a rub. In addition, the rub is suitable for fragile aged skin. The emulsion is suitable for improving fine lines, wrinkles, dryness reducing redness and irritation.

Percentages are examples only and it will be appreciated that any suitable percentage may be used depending on the use.

The emulsion is prepared in the following way: Phase A: disperse Ultrez 10 (carbomer) in water and let is swell for 20 minutes, then add phase B; heat to 75°C. Heat Phase C separately to 75°C. Mix the two phases under stirring, homogenise, add Phase D, neutralise with Phase E, cool until reaching 30°C, then add Phase F and Phase G; adjust to pH to 6 with ∼NaOH. It will be understood that this is an example only and any suitable method known in the art may be used.

In a further embodiment, the composition may comprise one or more of water, a carbomer, a sorbate such as potassium sorbate, a sugar alcohol, such as glycerine, an alcohol such as 2-(2-Ethoxyethoxy)ethanol, a polyoxyethylene stearyl ethers, such as Steareth 2, a fatty alcohol or phosphoric acid or a mixture of fatty alcohol and phosphoric acid , such as cetearyl alcohol, dicetyl phosphate and Cereth 10 phosphate or combinations thereof, a siloxane, such as cyclomethicone, a Caprylic Capric Triglycerides, a sorbitan Stearate, Parabens, Sodium hydroxide, an active agent such as a skin lightening agent, such as a mixture of lycerin (and) Butylene Glycol (and) Arcostaphylus Uva Ursi Leaf Extract (and) Mitracarpus Scaber Extract (ETIOLINE®), or a muscle health agent and peptide of the invention.

The following composition in Example 8 is an example of an emulsion or cream or a rub.

| **Ingredient** | **Exemplary (w/w)%** |
|---|---|
| Water Deionised | qs 100 |
| Carbomer | 0.10 |
| Potassium Sorbate | 0.10 |
| Transcutol | 3.00 |
| Glycerin | 8.00 |
| Volpo S2 [Steareth 2] | 0.60 |
| Crodafos CES [Cetearyl Alcohol (and) Dicetyl Phosphate (and) Ceteth 10 Phosphate] | 4.00 |
| | |
| DC 344 [Cyclomethicone] | 2.00 |
| Crodamol GTCC [Caprylic/Capric triglyceride] | 10.00 |
| Crill 3 [Sorbitan Stearate] | 1.60 |
| | |
| Mixed Parabens | 0.30 |
| | |
| Sodium Hydroxide 30% | 0.35 |
| Water Deionised | 3.50 |
| | |
| Peptide | 3.00 |
| Active | 3.00 |

The active can be ETIOLINE (R) [Glycerine (and) Etylene Glycol (and)Arcostaphylus uva leaf extract and Mitracarpus Scaber extract] ETIOLINE ® is a skin lightening ingredient sold by SEDERMA (WO 98/05299 of Nov. 19, 1996).

It will be understood that ETIOLINE can be replaced with any active agent, such as an agent that aids muscle health, development or recovery.

This formulation can be made according to the procedures generally outlined in Example 7. The composition may be an emulsion or cream or rub comprising one or more of water, a carbomer, such as Ultrez 10, a sugar alcohol, such as glycerine, an alcohol such as phenova (phenoxyethanol and mixed parabens), a fatty acid ester such as ethylhexyl palmitate, a fatty alcohol such as cetearyl alcohol, a lactic acid ester such as myristyl lactate, a sorbate such as polysorbate 20 and/or potassium sorbate, a polymeric emulsifier such as Acrylate (C10 -30 alkyl acrylate) and a cross polymer, a siloxane, such as cyclomethicone, sodium hydroxide, at least one active agent, such as a muscle health agent or an agent for the treatment of stretch marks, such as siegesbeckia Orientalis extract (Darutoside) and a peptide.

The following composition in Example 9 is an example of an emulsion or cream. In an embodiment, the emulsion or cream is an anti-stretch mark cream.

| **Ingredient** | **Exemplary (w/w)%** |
|---|---|
| **Part A** | |
| Water Deionised | qs 100 |
| Ultrez 10 [Carbomer] | 0.40 |
| | |
| **Part B** | |
| Glycerin | 5.00 |
| Phenova [Phenoxyethanol (and) Mixed Parabens] | 0.80 |
| | |
| **Part C** | |
| Crodamol OP [Ethylhexyl Palmitate] | 4.00 |
| Crodacol CS90 [Cetearyl alcohol] | 0.50 |
| Crodamol ML [Myristyl Lactate] | 0.30 |
| Crillet 1 [Polysorbate 20] | 1.00 |
| | |
| **Part D** | |
| Pemulen TR2 [Acrylates/C 10-30 Alkyl Acrylate (and) Crosspolymer] | 0.20 |
| | |
| DC 345 [Cyclomethicone] | 2.00 |
| | |
| **Part E** | 0.10 |
| Potassium Sorbate | |
| | |
| **Part F** | |
| Water | 6.00 |
| Sodium Hydroxide 38% | 0.60 |
| | |
| **Part G** | |
| peptide | 3.00 |
| Active | 2.00 |

The active can be Darutoside (Siegesbeckia Orientalis Extract). Darutoside is a molecule sold by SEDERMA for the treatment of stretch marks. It will be understood Darutoside can be replaced with any active agent, such as any agent that aids muscle health, development or recovery.

This emulsion or rub is prepared in the following way: Phase A disperse Ultrez 10 (carbomer) in water and let it swell for 20 minutes, then add phase B; heat to 75°C. Heat Phase C separately to 75°C. Mix the two phases under stirring, homogenise, add Phase D, neutralise with Phase E, cool until reaching 30°C., then add Phase F and Phase G, adjust pH to -6 with NaOH.

In an embodiment of the invention the composition may be an emulsion, cream or a gel, preferably a gel, comprising one or more of water, a carbomer, such as Ultrez 10, a sugar alcohol, such as glycerine, an alcohol such as phenova (phenoxyethanol and mixed parabens), a sorbate such as polysorbate 20 and/or potassium sorbate, a polymeric emulsifier such as Acrylate (C10 -30 alkyl acrylate), a siloxane, such as cyclomethicone, sodium hydroxide, a peptide and at least one agent such as an agent for muscle health, recovery or development, or a suitable moisturising agent, such as an agent comprising Imperata Cylindrica (root) extract, water, glycerine, PEG-8, and carbomer (MOIST 24). In an embodiment, the gel is a moisturising gel.

The following composition in Example 10 is an example of a gel. In an embodiment, the gel is a moisturising gel

| **Ingredient** | **Exemplary (w/w)%** |
|---|---|
| **Part A** | |
| Ultrez 10 [Carbomer] | 0.20 |
| Water Deionised | qs 100 |
| | |
| **Part B** | |
| Glycerin | 3.00 |
| Phenova [Phenoxyethanol (and) Mixed Parabens] | 0.80 |
| | |
| **Part C** | |
| Crillet 1 [Polysorbate 20] | 0.50 |
| | |
| **Part D** | |
| Potassium Sorbate | 0.10 |
| | |
| **Part E** | |
| | |
| Pemulen TR1 [Acrylates/C10-30 Alkyl | 0.20 |
| Acrylate Crosspolymer] | 3.00 |
| DC 345 [Cyclomethicone] | 2.00 |
| | |
| **Part F** | |
| | |
| Water | 4.00 |
| Sodium Hydroxide 38% | 0.40 |
| | |
| **Part G** | |
| | |
| Peptide | 3.00 |
| Active | 5.00 |
| MOIST-24 is a moisturising plant extract sold by SEDERMA (WO 01/62218 of Aug. 30, 2001). | |

This formulation can be made according to the procedures generally outlined in Example 11.

The active can be MOIST-24 (R) [Imperata Cylindrica (root) Extract (and) water (and) Glycerin (and) PEG-8 (and) Carbomer]. It will be understood that any agent can be used to replace MOIST-24. For example, any agent that aids muscle health, development or recovery.

In an embodiment of the invention the composition may be an emulsion, cream or rub comprising one or more of water, a carbomer, such as Ultrez 10, a sorbate such as polysorbate 20, polysorbate 60 and/or potassium sorbate, an alcohol such as phenova (phenoxyethanol and mixed parabens), butylene glycol (1,3-butanediol), lanolin alcohol, and/or cetearyl alcohol, sorbitan stearate, Polydimethylsiloxane such as dimethicone, an isotridetyl isononanoate, a caprylic/capric triglyceride, a cetyl ester, sodium hydroxide, water, an agent for muscle health, recovery and/or development, or an anti-aging agent, such as an agent comprising butylene glycol, water, laureth-3, hydroxyethylcellulose and acetyl-dipeptide-1-cetylester (CALMOSENSINE®).

The following composition in Example 11 is an example of a cream or a rub.

| **Ingredient** | **Exemplary (w/w)%** |
|---|---|
| Part A | |
| Water Deionised | qs 100 |
| Ultrez 10 [Carbomer] | 0.20 |
| | |
| Part B | |
| Potassium Sorbate | 0.10 |
| | |
| Part C | |
| | |
| Butylene Glycol | 2.00 |
| Phenova [Phenoxyethanol (and) Mixed Parabens] | 0.80 |
| | |
| Part D | |
| | |
| Crill 3 [Sorbitan Stearate] | 1.00 |
| Crillet 3 [Polysorbate 60] | 2.50 |
| DC 200 [Dimethicone | 2.50 |
| Crodamol TN [Isotridetyl Isononanoate] | 5.00 |
| Crodamol GTCC [Caprylic/Capric Triglyceride] | 5.00 |
| Crodamol SS [Cetyl Esters] | 1.00 |
| Super Hartolan [Lanolin Alcohol] | 0.50 |
| Super Sterol Ester [C10-C30 Cholesterol/ Lanosterol esters] | 0.30 |
| Crodacol CS90 [Cetearyl Alcohol] | 3.00 |
| | |
| Part E | |
| | 2.50 |
| Water Deionised | 0.25 |
| Sodium Hydroxide 38% | |
| | |
| Part F | |
| peptide | 3.00 |
| Active agent | 4.00 |

This formulation can be made according to the procedures generally outlined in Example 9. The active can be CALMOSENSINE (R) [Butylene Glycol (and) water (and) Laureth-3 (and) Hydroxyethylcellulose (and) Acetyl-Dipeptide-1-cetylester] Calmosensine(R) is an analgesic peptide offered by SEDERMA (WO 98/07744 of Feb. 26, 1998). It will be understood that any agent can be used to replace CALMOSENSINE. For example, any agent that aids muscle health, development or recovery.

In an embodiment of the invention the composition may be an emulsion, cream or rub comprising one or more of water, a carbomer, such as Ultrez 10, a sugar alcohol, such as glycerine, a sorbate such as potassium sorbate, a steareth such as Steareth 10, a fatty alcohol or phosphoric acid or a mixture of fatty alcohol and phosphoric acid, such as cetearyl alcohol, dicetyl phosphate and Cereth 10 phosphate or combinations thereof, diethyhexyl succinate, mixed parabens, Sorbitan Stearate, Sodium Hydroxide, water, a peptide, and an active agent, such as an agent for muscle health, recovery and/or development or an agent for mature skin, such as one comprising Trifolium Pratense(Clover) Flower Extract (and) Glycerin (and) Butylene Glycol (and) Lecithin (TEROCARE®).

The following composition in Example 12 is an example of a cream or a rub.

| **Ingredient** | **Exemplary (w/w) %** |
|---|---|
| Part A | |
| Ultrez 10 [Carbomer] | 0.20 |
| Water | qs 100 |
| | |
| Part B | |
| Glycerin | 3.50 |
| | |
| Part C | 0.10 |
| Potassium Sorbate | |
| Part D | |
| | 1.50 |
| Volpo S10 [Steareth 10] | 3.50 |
| Crodafos CES [Ceterayl Alcohol | |
| | |
| Dicetyl Phosphate(and) Ceteth-10 Phosphate] DC 200 | 2.00 |
| [Dimethicone] | 7.00 |
| Diethylhexyl Succinate | |
| | |
| Mixed Parabens | 0.30 |
| | |
| Crill 3 [Sorbitan Stearate] | 0.40 |
| | |
| | |
| Part E | |
| Sodium Hydroxide 38% | 0.20 |
| Water | 4.00 |
| | |
| | |
| Part F | |
| Active agent | 3.00 |
| Peptide | 3.00 |
| | |

This formulation can be made according to the procedures generally outlined in Example 9. The active agent can be STEROCARE (TM) [Trifolium Pratense (Clover) Flower Extract (and) Glycerin (and) Butylene Glycol (and) Lecithin Sterocare(R) is offered by SEDERMA as an active ingredient for mature skin (FR 2769502 of Apr. 14, 2000, WO 99/18927 of Apr. 22, 1999). It will be understood that any agent can be used to replace STEROCARE®. For example, any agent that aids muscle health, development or recovery.

The following composition in Example 13 is an example of a rub or a tonic.

| **Ingredient** | **Exemplary (w/w)%** |
|---|---|
| Part A | |
| Water deionised | qs 100 |
| | |
| Part B | |
| Mixed Parabens | 0.14 |
| Butylene Glycol | 2.00 |
| | |
| Part C | |
| Peptide | 0.0008 |
| Ethanol | 10.00 |
| | |
| Part D | |
| Crillet 1 (Polysorbate 20) | 1.50 |
| Fragrance | 0.10 |

The following composition in Example 14 is an example of a cream or a rub.

| **Ingredient** | **Exemplary (w/w) %** |
|---|---|
| Part A | |
| Ultrez 10 [Carbomer] | 0.20 |
| Water Deionised | qs 100 |
| | |
| Part B | |
| Glycerin | 3.00 |
| | |
| Part C | |
| Potassium Sorbate | 0.10 |
| | |
| Part D | |
| Volpo S10 [Steareth 10] | 1.50 |
| Crodafos CES [Ceterayl Alcohol Dicetyl Phosphate (and) Ceteth-10 Phosphate] | 3.00 |
| DC 200 [Dimethicone] | 2.00 |
| Crodamol OSU [Diethylhexyl Succinate] | 5.00 |
| Mixed Parabens | 0.30 |
| Crill 3 [Sorbitan Stearate] | 0.40 |
| | |
| Part E | |
| Sodium Hydroxide 38% | 0.20 |
| Water Deionised | 4.00 |
| Part F | |
| Water | 10.0 |
| Peptide | 0.00075 |
| Agent | 0.001 |
| Agent | 0.002 |

The agent can be Deferoxamine and/or Berberine. These are used as skin thickeners. Deferoxamine and Berberine can be replaced with another active agent, such as an agent for muscle health, recovery and development.

The following composition in Example 15 is an example of a gel or rub.

| **Ingredient** | **Exemplary (w/w)%** |
|---|---|
| Part A | |
| Water Deionised | qs 100 |
| | |
| Part B | |
| Butylene Glycol | 5.00 |
| Phenova [Phenoxyethanol (and) Mixed Parabens] | 0.80 |
| | |
| Part C | |
| Crill 3 [Sorbitan Stearate] | 1.20 |
| Crillet 3 [Polysorbate 60] | 3.00 |
| DC 200 [Dimethicone] | 2.00 |
| Crodamol IPM [Isopropyl Myristate] | 5.00 |
| Crodamol W [Stearyl Heptanoate] | 0.30 |
| Crodamol GTCC [Caprylic/Capric Triglyceride] | 5.00 |
| Crodacol CS90 [Cetearyl Alcohol] | 2.00 |
| | |
| Part D | |
| Carbopol 980 at 2% [Carbomer] | 10.00 |
| | |
| Part E | |
| Potassium Sorbate | 0.10 |
| Part F | |
| | |
| Water | 2.00 |
| Sodium Hydroxide | 0.20 |
| | |
| Part G | |
| | |
| Water | 10.0 |
| Peptide | 0.00045 |
| Active agent | 0.1 |
| Active agent | 0.0001 |

This gel can be prepared in the following way: Homogenize Part B and pour it into Part A. Heat Part (A+B) to 75°C. Heat Part C and Part D to 75°C. Pour Part C into Part (A+B) with helix stirring; then, pour Part D into Part (A+B+C). Add Part F and Part E. Pour Part G at about 35°C.

The active can be Rutin and or Bowman Birk Inhibitor. These agents are used for tissue regeneration. Rutin and Bowman Birk Inhibitor can be replaced with another active agent, such as an agent for muscle health, recovery and development.

### EXAMPLE 16

### Puromycin ELISA

### Study Description

Puromycin is an aminonucleoside antibiotic produced by the bacterium *Streptomyces alboniger.* It is a structural analogue of aminoacyl-transfer RNA and, as such, can be incorporated into elongating peptide chains via the formation of a peptide bond. However, whereas aminoacyl-tRNAs contain a hydrolyzable ester bond, puromycin hasn't it in the equivalent position. Thus, the binding of puromycin to a growing peptide chain prevents a new peptide bond from being formed with the next aminoacyl-tRNA. As a consequence, puromycin binding results in the termination of peptide elongation, and leads to the release of the truncated puromycin bound peptide from the ribosome. At very high concentrations, puromycin effectively shuts down the elongation phase of translation and thus inhibit protein synthesis; however, at very low concentrations, that do not inhibit the overall rate of translation, the rate at which puromycin-labelled peptides are formed reflects the overall rate of protein synthesis. This later property makes puromycin a potential tool for the measurement of changes in protein synthesis rates.

### Methodology

1. Dispense 100µl Coating Buffer per well in the required number of wells in a 96 well ELISA plate. Dilute and mix the Protein extract (in the same buffer the sample is in) to 5ug/µl. Immediately dispense 1µl /well into plate.
2. Incubate the plate at 4°C for 16-24 h.
3. Vigorously shake out contents. Wash 1x with 200ul PBS.
4. Add 200µl Blocking Solution (5% BSA in PBS). Incubate over the day 4-6 h at RT.
5. Dilute Puromycin antibody in Blocking solution and incubate overnight at 4°C.
8. Wash 2X with 200ul PBS
9. Add 100µl Secondary Antibody diluted 1:1000 in Blocking Soln. Incubate 1 h at RT.
10. Wash 4X 200µl PBS. Shake out contents vigorously.
11. Add 100µl TMB Substrate and incubate for 15-20 min
12. Add Stop solution and read the plate at 450nm.

### Results

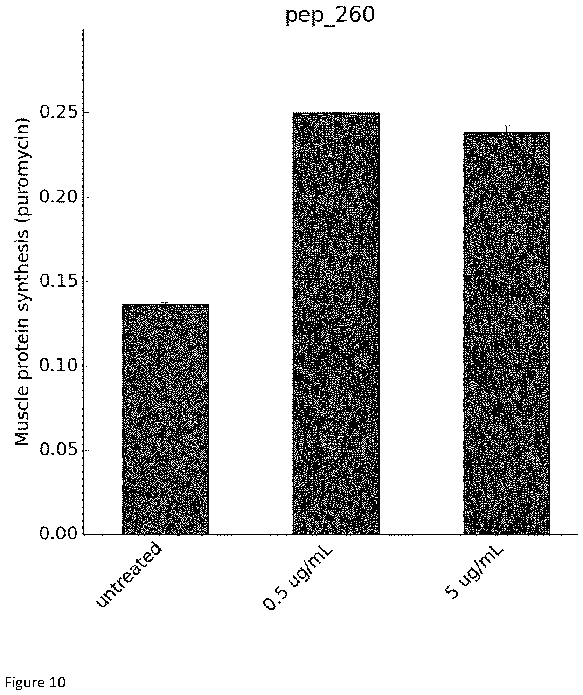

Figure 10 illustrates an increase in protein synthesis.

## Claims

1. A topical cosmetic composition comprising a cosmetically effective amount of a peptide comprising an amino acid sequence of SEQUENCE ID NO. 1 or a variant thereof.

2. The composition of Claim 1, wherein said peptide is a bioactive peptide.

3. The composition of Claim 1, wherein said variant is a bioactive variant.

4. The composition of any one of the preceding Claims comprising a plurality of peptides.

5. The composition of any one of the preceding Claims, wherein the composition further comprises at least one cosmetically acceptable excipient or additive.

6. The composition of any one of the preceding Claims, wherein composition further comprises at least one cosmetically acceptable active.

7. The composition of any one of the preceding Claims, wherein said variant or bioactive variant has from about 70% to about 99% sequence identity with SEQUENCE ID NO. 1.

8. The composition of any one of the preceding Claims, wherein said variant or bioactive variant has an amino acid sequence comprising any one of SEQUENCE ID NO. 2 to 85.

9. The composition of any one of the preceding Claims, wherein the peptide comprises from about 3 to about 50 amino acids in length.

10. The composition of any one of the preceding Claims, wherein the peptide or variant has one or more of anti-aging activity, cellular growth promoting activity, glucose transport-promoting activity, anabolic activity or protein synthesis activity.

11. Non-therapeutic use of the composition of any one of Claims 1 to 10 as a cosmetic.

12. Non-therapeutic use of the composition of any one of Claims 1 to 10 in slowing or inhibiting, or preventing aging of human skin.

13. Non-therapeutic use of the composition of any one of Claims 1 to 10 in improving muscle status in a mammal.

14. The use of Claim 13, wherein the muscle status is promoting recovery of muscle, typically following exercise, maintaining or restoring muscle health (for example lean tissue mass) in a mammal, and/or enhancing physical performance.

15. Non-therapeutic use of the composition of any one of the preceding Claims in promoting growth of tissue.

16. The use of Claim 15, wherein the growth of tissue is in promoting growth of epithelial tissue, promoting growth of skin, promoting growth of an organ, and/or promoting growth of an organism.

17. Non-therapeutic use of the composition of any one of the preceding Claims in muscle growth.

18. Non-therapeutic use of the composition of any one of the preceding Claims in increasing protein synthesis.

19. The composition of any one of Claims 1 to 10 or the use of any one of Claims 11 to 18, wherein the composition is in a formulation selected from the group comprising creams, multiple emulsions, anhydrous compositions, aqueous dispersions, oils, milks, balsams, foams, lotions, gels, cream gels, hydro-alcoholic solutions, hydro-glycolic solutions, cosmetic, personal care product, hydrogels, liniments, sera, soaps, dusting powder, paste, semi solid formulations, liniments, serums, shampoo, conditioner, ointments, any rinse off formulation, talc, mousses, powders, sprays, aerosols, solutions, suspensions, emulsions, syrups, elixirs, polysaccharide films, patches, gel patches, bandages, an adhesive system, water-in-oil emulsions, oil-in-water emulsions, and silicone emulsions.

20. A medical device comprising the composition of any one of Claims 1 to 10 or 19.
